# EUROPEAN PATENT SPECIFICATION

(11) **EP 1 768 608 B1**
(45) Date of publication and mention of the grant of the patent: **13.06.2012**
(21) Application number: 05753417.4
(22) Date of filing: 24.05.2005
(51) Int. Cl.: A61F 2/84

(54) **BIFURCATED STENT DELIVERY SYSTEM**
GEGABELTES STENT-ABGABESYSTEM
SYSTEME DE MISE EN PLACE D'ENDOPROTHESE VASCULAIRE BIFURQUEE

(30) Priority: 08.06.2004 US 863724
(43) Date of publication of application: 04.04.2007
(73) Proprietor: Boston Scientific Limited, an Irish company, Barbados, West Indies (BB)
(72) Inventor: TRAN, Thomas Trinh, Coon Rapids, MN 55433 (US); EDENSCHINK, Tracee, Wayzata, MN 55391 (US); WEBER, Jan, Maple Grove, MN 55311 (US)
(74) Representative: Vossius & Partner
(86) International application number: PCT/US2005/018235
(87) International publication number: WO 2005/122958

(56) References cited:
- WO-A-03/017872
- US-A- 5 776 141
- US-A- 5 843 027
- US-A1- 2002 072 755

## Description

A stent delivery system employing a stent assembly with branches intended for deployment in the adjacent branches of a vessel bifurcation has been proposed to allow placement of a portion of the assembly in both a primary passage, such as an artery, and a secondary passage, such as a side branch artery. Additionally, these stents generally have an opening which allows for unimpeded blood flow into the side branch artery. However, problems are still encountered in orienting the stent relative to the side branch at the bifurcation of the primary and secondary passages. Moreover, such bifurcated assemblies are typically specially manufactured at an increased cost over a more standard stent intended for single vessel deployment.

In delivering a stent to a vessel location, many current devices rely on either passive torque (e.g., pushing the stent forward and allowing the stent that is fixed on the guidewire/balloon to passively rotate itself into place) or creating torque from outside of the patient to properly orient the medical device in the passage. These devices and methods of achieving proper angular orientation have not been shown to be effective in properly placing and positioning the stent.
US 5,776,141 A discloses a catheter for use in combination with a balloon angioplasty catheter for delivering stents and other intraluminal prostheses. The catheter comprises a tubular catheter body having a radially expansible portion. The stent is disposed over the radially expansible portion, and structures is provided for retaining the stent on the tubular body prior to deployment. The retaining structure may be active or passive.

Thus, a need exists to provide a catheter which is capable of allowing a medical device such as a stent to be easily maneuvered and aligned at a vessel bifurcation or other location, while also adequately protecting the catheter and/or balloon to which the stent is mounted. The present invention addresses this need by providing a catheter system with a rotatable sheath apparatus which a stent may be mounted on or engaged to. The rotatable assembly is rotatable about the catheter shaft thereby eliminating the need to apply torque to the catheter shaft to align the stent at a vessel bifurcation. The stent delivery system of the present invention is defined by the features of the claims.

Without limiting the scope of the invention a brief summary of some of the claimed embodiments of the invention is set forth below. Additional details of the summarized embodiments of the invention and/or additional embodiments of the invention may be found in the detailed description of the invention below.

Catheter systems for delivery of multiple stents or stent segments, wherein at least one of the stents is mounted on the catheter with a freely rotating deployment sheath and assembly are described in U.S. Patent Application No. 10/375,689, filed February 27, 2003 and U.S. Patent Application No. 10/657,472, filed September 8, 2003 both of which are entitled *Rotating Balloon Expandable Sheath Bifurcation Delivery*; U.S. Patent Application No. 10/747,546, filed December 29,2003 and entitled *Rotating Balloon Expandable Sheath Bifurcation Delivery System*; U.S. Patent Application No. 10/757,646, filed January 13, 2004 and entitled *Bifurcated Stent Delivery System*; and U.S. Patent Application No. 10/84,337, filed February 23, 2004 and entitled *Apparatus and Method for Crimping a Stent Assembly*.

As used herein the term 'stent' refers to an expandable prosthesis for implantation into a body lumen or vessel and includes devices such as stents, grafts, stent-grafts, vena cava filters, etc. In some embodiments a stent may be at least partially constructed of any of a variety of materials such as stainless steel, nickel, titanium, nitinol, platinum, gold, chrome, cobalt, as well as any other metals and their combinations or alloys. A stent may be at least partially constructed of a polymer material. A stent may be at least partially constructed of a shape-memory polymer or material. A stent may be balloon expandable, self-expandable, hybrid expandable or a combination thereof. In some embodiments a stent may include one or more areas, bands, coatings, members etc that is (are) detectable by imaging modalities such as X-Ray, MRI or ultrasound. In some embodiments at least a portion of the stent is at least partially radiopaque. In some embodiments a stent may include one or more therapeutic and/or lubricious coatings applied thereto.

Some embodiments of the present invention are directed to such catheter systems and rotating assemblies wherein the catheter is a balloon catheter having a balloon at least partially constructed of a compliant material and at least one rotatable sheath or sheath section at least partially disposed thereabout which is at least partially constructed of a non-compliant material and/or composite material.

At least one stent is disposed about the at least one sheath or sheath section prior to delivery. A guidewire is moveably engaged to the rotatable sheath in order to allow the rotatable sheath to rotatingly align the stent or stents at a vessel bifurcation. In some embodiments the guidewire extends between the stent and sheath exiting radially from a guidewire hole in the wall of the sheath and/or a secondary opening in the stent.

In at least one embodiment the catheter system employs a guidewire housing through which the guidewire is passed. The guidewire housing is fixedly engaged to the rotatable sheath and the stent is disposed thereabout. In some embodiments the guidewire housing extends through the secondary opening of the stent whereupon the guidewire exits the guidewire housing. In some embodiments the guidewire extends from a region of the rotatable sheath proximal to the stent to a distal region and/or distal end of the stent.

In at least one embodiment the guidewire housing has a length of which a majority of is engaged to the rotatable sheath. In some embodiments the entire length of the guidewire housing is engaged to the rotatable sheath. The guidewire housing may be integral with the rotatable sheath, be chemically or adhesively bonded to the rotatable sheath, fused, welded or otherwise engaged to the rotatable sheath.

In at least one embodiment the guidewire housing is constructed at least partially of one or more flexible materials such as Polyisobutylene, Polyurethane, silicone rubber; other synthetic rubbers such as SBS (Stryrene Butadiene), SEBS and SIS, latex, etc. In some embodiments at least a portion of the guidewire housing is constructed of a hypotube of nitinol or other metal or alloy which defines one or more substantially spiral shaped cuts or grooves therethrough.

In at least one embodiment a rotatable sheath extends over at least a portion of the balloon and at least a portion of the catheter shaft proximally adjacent thereto. In some embodiments the rotatable sheath has a plurality of longitudinal sections. For example, in at least one embodiment a rotatable sheath has three sections. A first section of a first flexural modulus value, a second section of a second flexural modulus value and a third section of a third flexural modulus value. The first or distal most section is positioned substantially about the balloon and may have a length approximately the same as that of the balloon. The second section is proximally adjacent the first section and the third section is proximally adjacent the second section. The second section and/or third section has/have a different flexural modulus value than that of the first section. In some embodiments the second flexural modulus value is greater than that of the first flexural modulus value but less than the third flexural modulus

In at least one embodiment the rotatable sheath is has a uniform material construction but is provided with sections of differing stiffness and/or flexural modulus by having the wall of the sheath be of varied thickness: providing one or more section of wall with a braided structure, while providing others with different braid or non-braided configurations; providing sections with one or multi-layer construction, pre-stretching one or more layers; selectively ablating or otherwise removing material from one or more layers; etc.

In at least one embodiment for example, a first section of the sheath proximally adjacent to the balloon may have a wall thickness greater than that of a second section of the sheath disposed about the balloon. In some embodiments a region of the sheath wall between the first and second sections may have a tapered thickness.

In at least one embodiment a guidewire underlies at least a portion of the at least one sheath. In some embodiments the guidewire passes through a guidewire opening defined by the wall of the at least one sheath.

In at least one embodiment a first sheath is rotatably disposed about a proximal or first section of the balloon and a second sheath is disposed about a distal or second section of the balloon. The second sheath may be rotatable or non-rotatable about the balloon. In some embodiments a first stent is disposed about the first sheath and a second stent is disposed about the second sheath prior to delivery of the stents. In some embodiments the first sheath and the second sheath at least partially overlap one another. In some embodiments the first sheath and the second sheath are longitudinally spaced apart from one another and define a gap or space therebetween. In some embodiments both the first sheath and the second sheath are at least partially constructed of a non-compliant material. In some embodiments the first sheath has a greater diameter than the second sheath. In some embodiments the first sheath is more compliant than the second sheath.

In at least one embodiment a first sheath is disposed about the balloon. The first sheath having a length at least as great as that of the balloon. A second sheath is rotatably disposed about a distal portion of the first sheath. In some embodiments the distal portion of the first sheath is more or less compliant than the remaining portion(s) of the first sheath. In some embodiments the distal portion of the first sheath defines a plurality of openings or slits wherein the respective areas of the wall of the first sheath have been cut, removed or thinned.

In at least one embodiment a non-compliant sheath is rotatably disposed about the relatively compliant balloon of the catheter. The sheath is provided with a less compliant region in the sheath wall or the sheath is provided a region of the wall having an aneurysm shape. When the non-compliant balloon is expanded the less compliant or aneurysm shaped region of the relatively non-compliant sheath will be pushed or shaped in a radially outward direction to a greater extent than the rest of sheath. In some embodiments a stent having a secondary branch opening defined by a plurality of extension members or fingers is disposed about the sheath, such that when the balloon is expanded the less compliant or aneurysm shaped region of the relatively non-compliant sheath pushes the fingers outward into a branch of a vessel bifurcation.

A detailed description of the invention is hereafter described with specific reference being made to the drawings.
FIG. 1 is a side view of a rotating sheath assembly.
FIG. 2 is a side view of the assembly shown in FIG. 1 shown configured for delivery of a stent.
FIG. 3 is a side view of a stent delivery system. The stent delivery system is provided with a rotating collar.
FIG. 4 is a side view of the stent delivery system of FIG. 3 with the rotating sheath assembly and stent of FIG. 2 mounted thereon.
FIG. 5 is a side view of the stent delivery system of FIG. 4 shown being advanced along a guidewire to a vessel bifurcation prior to delivery of the stent.
FIG. 6 is a side perspective view of a stent, such as that shown in FIG. 2.
FIG. 7 is a side perspective view of the stent shown in FIG. 6 wherein a side branch opening is shown formed.
FIG. 8 is a cross-sectional view of the stent of FIG. 7.
FIG. 9 is a side view of the stent depicted in FIG. 5, wherein the stent has been delivered from the stent delivery system, by balloon expansion and the assembly subsequently withdrawn from the vessel(s).
FIG. 10 is a side view of an embodiment of the invention wherein the stent delivery system is provided with a rotatable sheath having differing characteristics along at least part of its length.
FIG. 11 is a side view of an embodiment of the invention wherein the stent delivery system is provided with a rotatable sheath wherein a portion of the sheath wall has a stepped thickness.
FIG. 12 is a side view of an embodiment of the invention wherein the stent delivery system is provided with a rotatable sheath wherein a portion of the sheath wall has a tapered thickness.
FIG. 13 is a cross-sectional view of an embodiment of the invention wherein the stent delivery system is provided with a secondary guidewire housing that is engaged to at least a portion of the rotatable sheath.
FIG. 14 is a cross-sectional view of an embodiment of the invention wherein the stent delivery system is provided with a secondary guidewire housing that is integral with the wall of the rotatable sheath.
FIG. 15A is a perspective view of an embodiment of the invention wherein the balloon and the rotatable sheath of the stent delivery system are shown in the unexpanded state.
FIG. 15B is a perspective view of the embodiment shown in FIG. 15A in the expanded state.
FIG. 16 is a cross-sectional view of the embodiment shown in FIG. 15A.
FIG. 17 is a perspective view of an embodiment of the invention wherein the stent delivery system is shown prior to delivery and is provided with a proximal rotatable sheath and a distal sheath.
FIG. 18 is a perspective view of the embodiment shown in FIG 17 wherein the balloon is shown in the expanded state during delivery of the stent(s).
FIG. 19 is a perspective view of an embodiment of the invention wherein the stent is shown prior to delivery and the proximal sheath and the distal sheath are configured to partially overlap.
FIG. 20 is a perspective view of the embodiment shown in FIG. 19, wherein the stent is shown in the expanded state.
FIG. 21 is a side view of a first configuration of the sheaths shown in FIG. 19.
FIG. 22 is a side view of a second configuration of the sheaths shown in FIG. 19.
FIG. 23 is a perspective view of an embodiment of the invention wherein the stent delivery system is shown prior to delivery and has a first sheath disposed about the balloon and a proximal rotatable second sheath disposed about the first sheath.
FIG. 24 is a perspective view of an embodiment of the invention illustrated in FIG. 23 shown during delivery of a stent(s).
FIG. 25 is a perspective view of an embodiment of the invention wherein the system is shown configured to expand a crown region of a stent, by pushing the sheath radially outward during balloon expansion to deploy the fingers of the crown region into a side branch of a vessel bifurcation.
FIG. 26 is a partial perspective view of the embodiments shown in FIG. 25 wherein the crown region is depicted prior to delivery.

While this invention may be embodied in many different forms, there are described in detail herein specific embodiments of the invention. This description is an exemplification of the principles of the invention and is not intended to limit the invention to the particular embodiments illustrated.

For the purposes of this disclosure, like reference numerals in the figures shall refer to like features unless otherwise indicated.

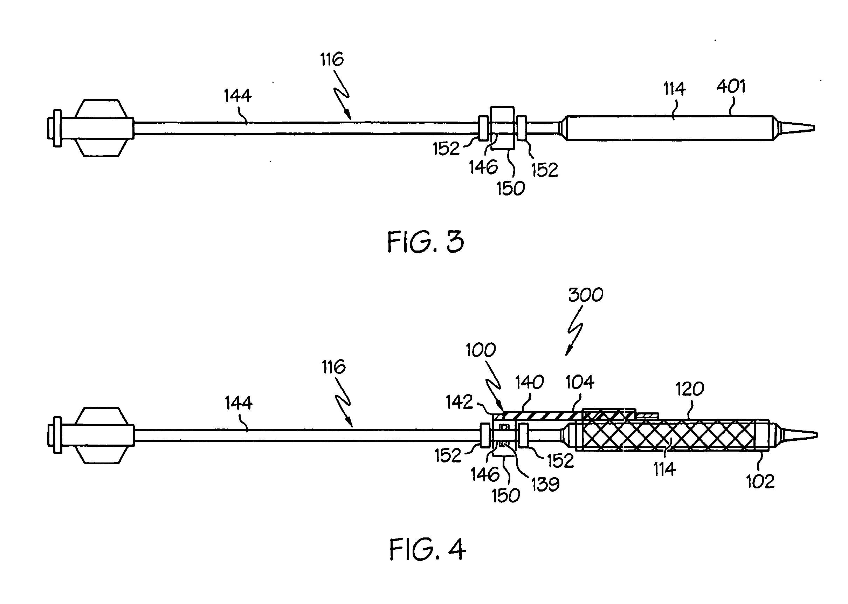

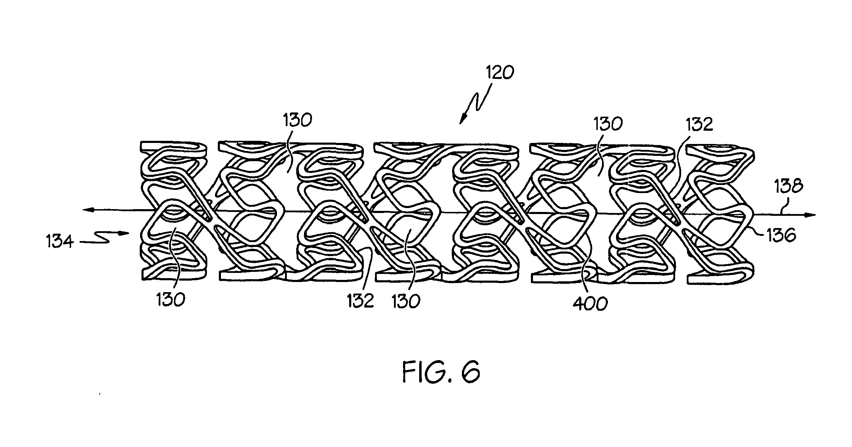

Referring now to the drawings which are for the purposes of illustrating embodiments of the invention only and not for purposes of limiting same, FIG s. 1-2 illustrate a an assembly 100 for use in a stent delivery system 300 which is mounted on a catheter body 116, such as is depicted in FIGs. 3-5, to provide the system with a rotating region that allows a stent 120, such as is shown in FIGs 6-9, to be properly aligned in a vessel bifurcation. Some additional examples of such assemblies are shown and described in U.S. Patent Application No. 10/375,689, filed February 27, 2003 and U.S. Patent Application No. 10/657,472, filed September 8, 2003 both of which are entitled *Rotating Balloon Expandable Sheath Bifurcation Delivery*; U.S. Patent Application No. 10/747,546, filed December 29, 2003 and entitled *Rotating Balloon Expandable Sheath Bifurcation Delivery System*; and U.S. Patent Application No. 10/57,646, filed January 13, 2004 and entitled *Bifurcated Stent Delivery System*.

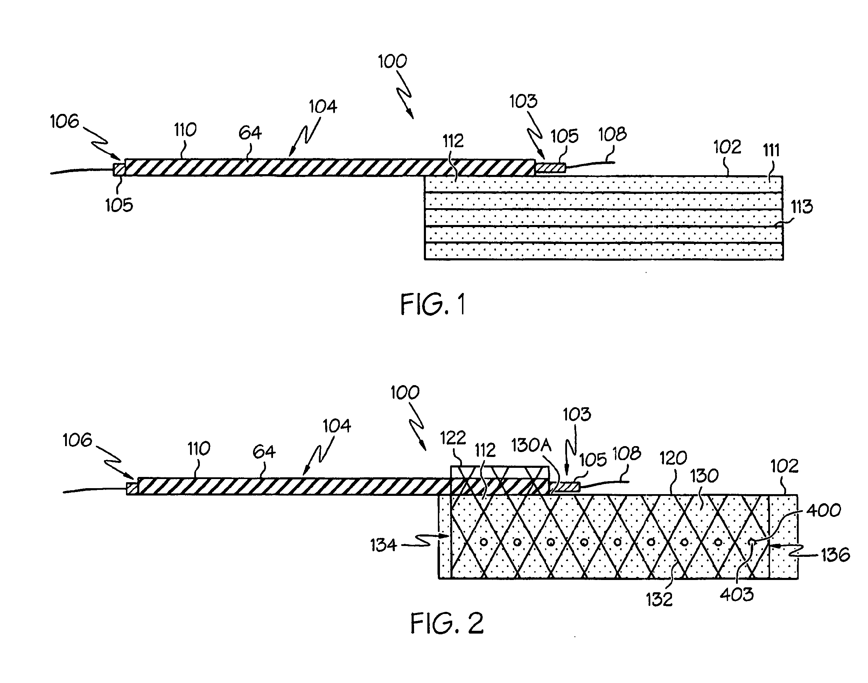

The rotating sheath assembly 100 depicted in FIGs. 1-2 comprises a tubular sleeve or sheath 102 and a positioning or secondary guidewire housing 104. The housing 104 defines a secondary guidewire lumen 106 through which a secondary guidewire 108 may be passed.

Though the housing 104 may be constructed of a wide variety of materials including metal plastic, etc., in some instances the housing 104 may be an external reinforcing member or hypotube 64.

The hypotube 64 may comprise stainless steel, nitinol, one or more polymer materials or other material. To improve flexibility, in some cases the housing 104 is provided with one or more openings 110 along its length. For example, the housing 104 may be spiral cut to provide at least a continuous opening 110 which acts to provide improve the flexibility of the housing 104.

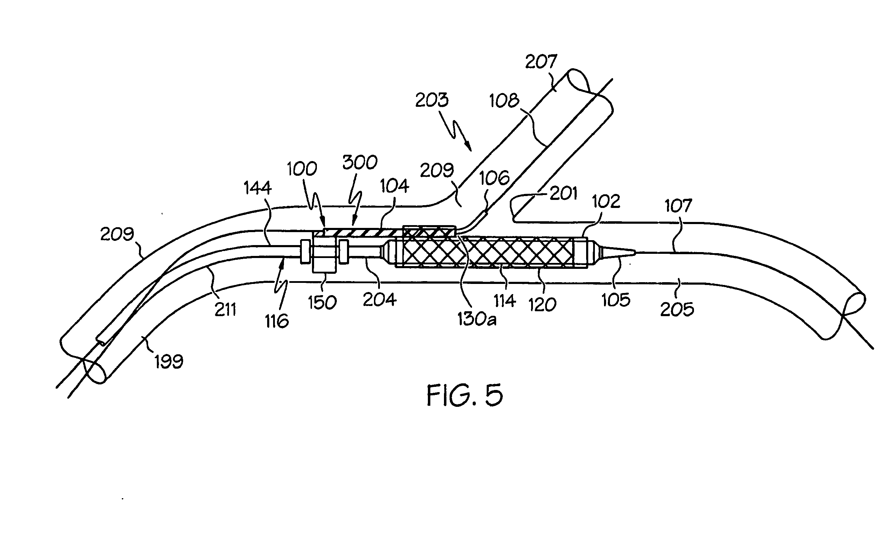

The assembly 100 includes a secondary guidewire housing 104 which further comprises an inner shaft 103, about which the hypotube 64 is disposed. The inner shaft 103 may be a flexible hollow tubular member which extends distally beyond the distal end of the hypotube 64. This distal and/or proximal tips 105 of the inner shaft 103 provides the housing with a flexible protective sheath about the guidewire 108 as it passes out of the secondary guidewire lumen 106. Such a protective covering prevents the guidewire 108 from excessively rubbing against the wall 201 of the vessel 199, such as in the manner depicted in FIG. 5; even where the secondary guidewire 108 exits the secondary lumen 106 at a significant angle. The inner shaft 103 may be constructed of any of a variety of flexible materials such as: PEBAX^{®}, nylon, urethane, and/or other materials in a single layer, multi-layer and/or braided configuration.

In many catheters, the shaft 144 of the catheter 116 defines a primary guidewire housing 211 through which a primary guidewire 107 may be advanced. In use, guidewires 107 and 108 are passed through a lumen or other body vessel 209 to a bifurcation 203. Primary guidewire 107 is then advanced into a primary branch of passage 205 of the bifurcation 203 while the secondary guidewire 108 is advanced into the adjacent or secondary branch 207 of the bifurcation 203. As the system is advanced along both guidewires 107 and 108, as a result of the divergent paths defined by the guidewires 107 and 108, the rotatable sleeve 104 will rotate the stent 120 into a desired position so that the secondary opening 130a of the stent is aligned with the secondary passage 207. Where the catheter 116 is a fixed wire system, the use of the primary guidewire is unnecessary.

Examples of the rotating assembly 100 include a distal portion of the housing 104 being engaged to at least a proximal portion of the sheath 102 at an engagement site 112. The manner or mechanism of engagement between the sheath and housing 104 may be by bonding, welding, adhering adhesively engaging, mechanically engaging or otherwise connecting the surfaces of the respective sheath 102 and housing 104.

The sheath 102 is a hollow tube of sheath material that is configured to be placed over the balloon 114 or other region of a catheter 116, such as in the manner illustrated in FIGs. 3 and 4. The sheath 102 is further configured to be rotatable about the catheter shaft and/or balloon 114, even when a stent 120 has been positioned about and/or affixed to the sheath 102.

In order to ensure that the sheath 102 is rotatable about a balloon 114 and/or other region of a catheter, even with a stent 120 crimped on to the sheath 102 and the catheter is being advanced through the a body, the sheath 102 may be constructed of a variety of low friction materials such as PTFE, HDPE, etc. In at least one embodiment the sheath 102 is at least partially constructed of a hydrophilic material, such as hydrophilic polymers such as; TECOPHILIC^{®} material available from Thermedics Polymer Products, a division of VIASYS Healthcare of Wilmington, Massachusetts; TECOTHANE^{®}, also available from Thermedics Polymer Products; hydrophilic polyurethanes, and/or aliphatic, polyether-based thermoplastic hydrophilic polyurethane; and any other material that provides the sheath 102 with the ability to rotate freely about the balloon 114 when in the "wet" state, such as when the catheter is exposed to body fluids during advancement through a vessel. Suitable sheath materials may also provide the sheath with rotatability in the "dry", or pre-insertion, state, but with the application of a greater amount of force than when in the wet state, such materials are referred to herein as being tecophilic.

A sheath 102 at least partially constructed from tecophilic material provides the sheath 102 with the ability to rotate freely about the balloon 114 when in the "wet" state, such as when the catheter is exposed to body fluids during advancement through a vessel. The tecophilic sheath 102 is also capable of rotation in the "dry", or pre-insertion, state, but with the application of a greater amount of force than when in the wet state.

In some cases the sheath 102 may be constructed of one or multiple materials, in one or more layers. For example, the sheath 102 may comprise an outer layer of a softer material than that of the material used in constructing an inner layer, such as has been previously described. In some embodiments, an example of which is shown in FIG. 1, the sheath 102 may be comprised of a matrix of a first material 111 and have one or more supportive stripes, strands, members or areas of a second supportive material 113 within, external to or internal to such a matrix.

The composition of the sheath 102 material, whether a single, multiple layer or stripe reinforced extrusion may include essentially any appropriate polymer or other suitable materials. Some example of suitable polymers include Hydrophilic Polyurethanes, Aromatic Polyurethanes, Polycarbonate base Aliphatic Polyurethanes, Engineering polyurethane, Elastomeric polyamides, block polyamide/ethers, polyether block amide (PEBA, for example available under the trade name PEBAX), and Silicones, Polyether-ester (for example a polyether-ester elastomer such as Arnitel available from DSM Engineering Plastics), Polyester (for example a polyester elastomer such as Hytrel^{®} available from Du Pont), or linear low density polyethylene (for example Rexell^{®}).

Example of suitable reinforcing materials whether alone or blended with other materials, mixtures or combination or copolymers include all Polyamides (for example, Durethan^{®} available from Bayer or Cristamid^{®} available from ELF Atochem), polyethylene (PE). Marlex high-density polyethylene, polyetheretherketone (PEEK), polyimide (PI), and polyetherimide (PEI), liquid crystal polymers (LCP), and Acetal (Delrin^{®} or Celcon^{®}).

Often the inner surface of the sheath 102 or the outer surface of the balloon 114 may include a coating of one or more low friction materials or include one or more low friction materials in its construction. Such a coating 401 is shown in FIG. 3 on the surface of the balloon 114 before assembly 100 has been placed thereabout, such as is depicted in FIG. 4. Coating 401 may however be placed between the balloon 114 and sheath 102 at any time. Some examples of a suitable coating material include but are not limited to: hydrogel, silicon, and/or BIOSLIDE^{®} available from SciMed Life Systems, Inc. of Maple Grove Minnesota.

As mentioned above, the sheath 102 is configured to be freely rotatable about a balloon of a catheter even when a stent 120, such as is shown in FIGs. 2 and 4 is crimped onto the sheath 102. When properly positioned on the sheath 102, a proximal portion 122 of the stent 120 is also disposed about at least a portion of the secondary guidewire housing 104. When properly positioned about the sheath 102 and the housing 104, at least a portion of the housing 104 and/or the secondary guidewire 108 extends distally through a cell opening 130 of the stent 120.

Stent 120 may be a stent, such as is shown in FIG. 6, which is at least partially constructed of a plurality of interconnected struts, connectors or members 132. The stent 132 defines a proximal opening 134, a distal opening 136 and a flow path 138 therebetween. The cell openings 130 are in fluid communication with the flow path 138.

When the secondary guidewire 108 and/or the secondary guidewire housing 104 is threaded through one of the cell openings 130 when the stent is positioned onto the assembly 100, such as is shown in FIGs. 2 and 4, the members 132 that define the selected cell opening 130a, as well as the shape of the opening 130a through which the secondary guidewire 108 exits the stent, may be distorted or modified in order to accommodate the passage of secondary guidewire 108 and/or the secondary guidewire housing 104 therethrough.

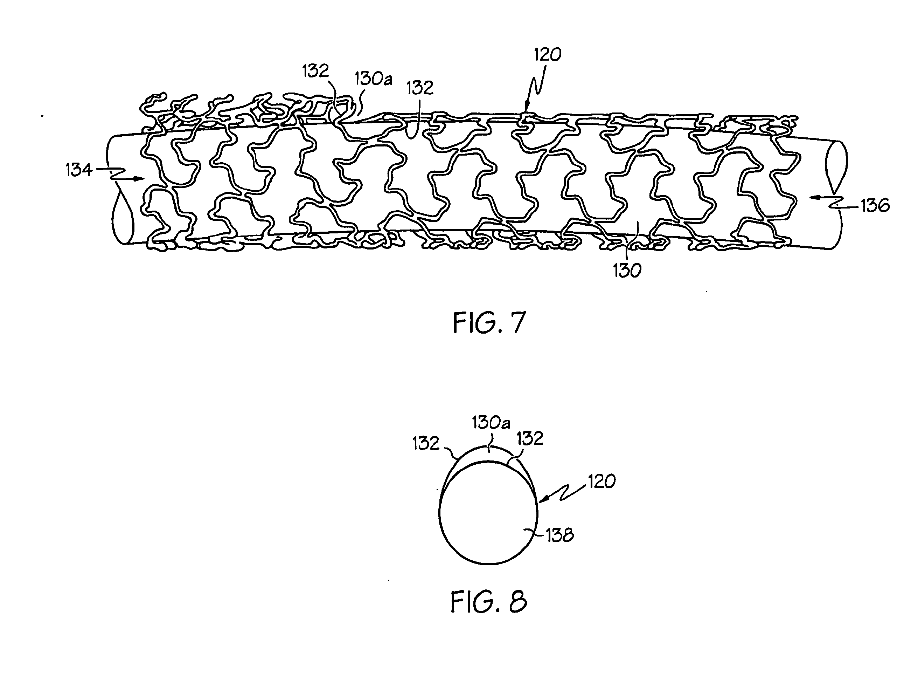

The modified cell opening 130a, hereinafter referred to as secondary opening 130a, is positioned on the stent 120 between the proximal opening 134 and the distal opening 136. The manner in which the secondary opening 130a, the members 132 adjacent thereto, and to an extent the stent 120 itself, are modified or distorted by the position of the secondary guidewire and/or secondary guidewire housing is depicted in FIGs 7 and 8.

It should be noted that when the stent 120 is placed on the assembly in the manner described above, the distortion of the secondary opening 130a and the adjacent members 132 is of a minimal extent, and is provide only to allow sliding passage of the secondary guidewire 108, and if desired a distal portion of the secondary guidewire housing 104, through the secondary opening 130a. As such, the actual size of the secondary opening 130a may be substantially similar, or only marginally different than that of the surrounding cell openings 130.

It should also be further noted that while stent 120 may be a standard "single vessel" stent that is provided with a secondary opening 130a in the manner described above, the stent 120 may also be a bifurcated stent having a trunk or stem portion, with one or more leg portions and/or branch openings adjacent thereto, through one of which the secondary guidewire may be passed. Such bifurcated stents and stent assemblies are well known in the art.

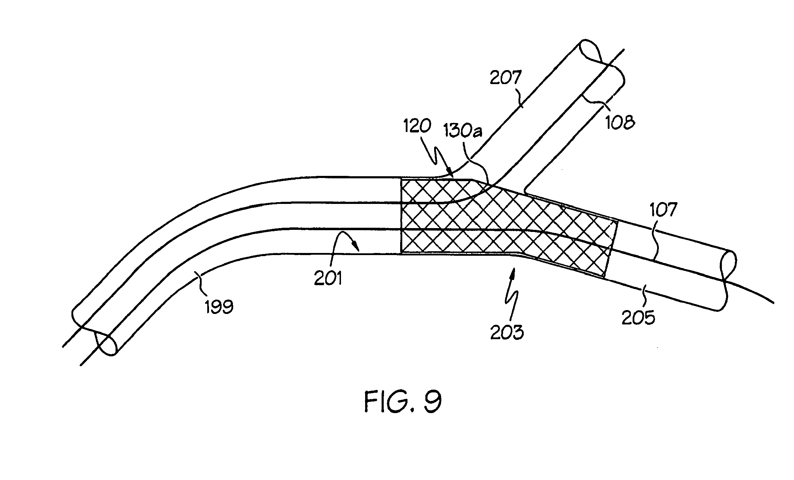

In some cases, the stent 120, sheath 102 or one or more portions thereof, may be configured to deliver one or more therapeutic agents to a delivery site such as within the vessel 199 or one or more areas adjacent thereto, such as shown in FIGs. 5 and 9.

To better accommodate placement of a therapeutic agent on the stent 120, in some instances one or more stent members 132, such as is shown in FIG. 6, maybe configured to include one or more holes, notches, or other surface features to which one or more therapeutic agents 400 may be placed for delivery to the aneurysm site. A therapeutic agent may be placed on the stent in the form of a coating. Often the coating includes at least one therapeutic agent and at least one polymer.

In at least one embodiment, an example of which is shown in FIG. 2, the sheath 102 may include one or more holes, notches, pores, cavities or other surface features 403 wherein one or more therapeutic agents 400 may be positioned. During expansion of the stent 120 the corresponding expansion of the sheath 102 may squeeze or otherwise act to release the agent 400 onto the stent and/or body.

A therapeutic agent may be a drug or other pharmaceutical product such as non-genetic agents, genetic agents, cellular material, etc. Some examples of suitable non-genetic therapeutic agents include but are not limited to: anti-thrombogenic agents such as heparin, heparin derivatives, vascular cell growth promoters, growth factor inhibitors, Paclitaxel, etc. Where an agent includes a genetic therapeutic agent, such a genetic agent may include but is not limited to: DNA, RNA and their respective derivatives and/or components; hedgehog proteins, etc. Where a therapeutic includes cellular material, the cellular material may include but is not limited to: cells of human origin and/or non-human origin as well as their respective components and/or derivatives thereof. Where the therapeutic agent includes a polymer agent, the agent may be a polystyrene-polyisobutylene-polystyrene triblock copolymer (SIBS), polyethylene oxide, silicone rubber and/or any other suitable substrate.

Once the stent 120 is positioned on the assembly 100, such as in the manner shown in FIG. 2, the assembly 100 may be slid onto a catheter 116, such as is shown in FIGs 3-4 so that the sheath 102 is rotatingly disposed about the balloon 114 and a proximal portion 140 of the secondary guidewire housing 104 may be engaged to an optional rotating collar 150. The use of collar 150 provides additional securement of the housing 104 to the catheter 116 as well as to minimize longitudinal displacement of the assembly relative to the balloon 114 in the manner described below.

The collar 150 is engaged to the proximal portion 140 of the secondary guidewire housing 104 by any engagement mechanism desired, such as welding, bonding, mechanical engagement, adhesive engagement, etc. As shown in FIG. 4 for example, the proximal portion 140 of the secondary guidewire housing 104 and the collar 150 are engaged externally at engagement site 142. Alternatively, the secondary guidewire housing 104 may be passed at least partially through the collar 150, and/or the collar 150 may define a lumen through which the secondary guidewire 108 may be passed before entering into the secondary guidewire housing 104.

Collar 150 may be a substantially cylindrical member that is disposed about the shaft 144 of the catheter 116 at a position proximal of the balloon 114. The collar 150 may be characterized as defining a catheter shaft lumen 146 through which the catheter shaft 144 is passed. In order to provide the collar 150 with the ability to freely rotate about the catheter shaft 144, the collar 150 defines a catheter shaft lumen 146 which has a diameter greater than the outer diameter of the shaft 144. In some embodiments one or more lubricious substances may be placed between the collar 150 and the shaft 144 to further encourage free rotation therebetween.

While the rotating collar 150 is free to rotate about the shaft 144, in some embodiments it will also be capable of being longitudinally displaced along the shaft 144 as well. As such, in some embodiments one or more locks or hubs 152 may be affixed about the shaft 144 on one or both sides of the collar 150 to prevent or limit the potential longitudinal displacement of the collar 150 relative to the shaft 144. In some embodiments the use of hubs 152 may be avoided or supplemented by providing the catheter shaft 144 with an annular protrusion or ring 139 which the collar 150 may be disposed about to prevent the assembly 100 from experiencing substantial longitudinal migration.

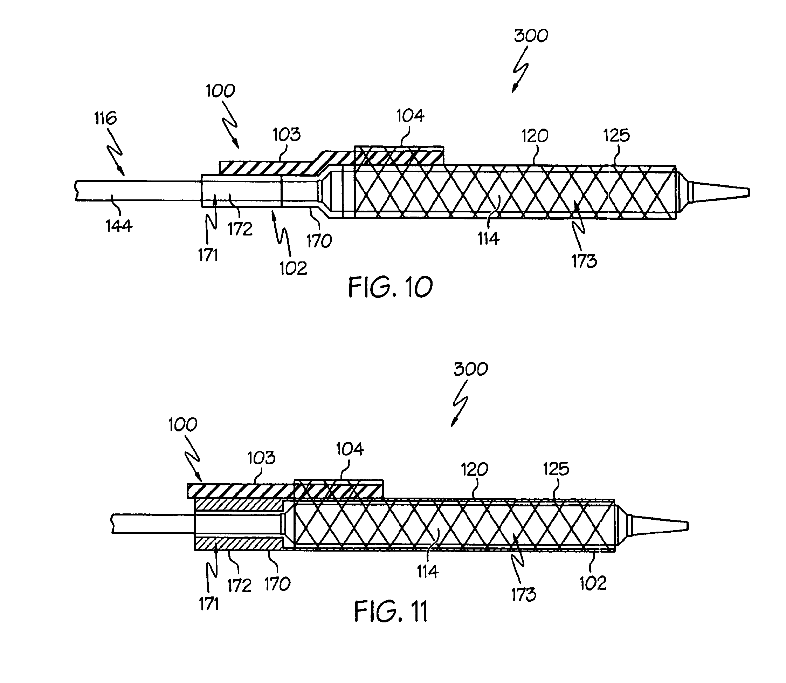

In at least one embodiment, an example of which is shown in FIG. 10, the sheath 102 may be configured to extend proximally beyond the proximal end of the balloon 114 and along a predetermined length of the catheter shaft 144. The length of the sheath 102 while less than that of the length of the catheter shaft 144 may otherwise be of any length desired.

In order to maintain flexibility and trackability of the catheter 116 the sheath 102 may be constructed to include a proximal region 171 that is less flexible, stiffer, and or harder than that of the distal region 173.

In the embodiment shown in FIG. 10 the distal region 173 of the rotatable sheath 102 is disposed about the balloon 114. In at least one embodiment the distal region 173 is at least partially constructed of a material having a lower flexural modulus value than that of the proximal region 171.

In some embodiments the distal region 173 has a flexural modulus value higher than that of the proximal region 171.

Where the proximal region 171 is stiffer than the distal region 173, the proximal region 171 will typically be constructed of material or materials having flexural modulus value(s) of about 300 MPa or more, where as the distal region 173 is constructed of a material or materials having a flexural modulus of about 300MPa or less. As indicated the regions 173 and 171 may be made stiffer or less stiff as desired, and may likewise be constructed of materials having any of a variety of flexural modulus values.

In some embodiments the proximal region 171 may have multiple sections having different flexural modulus values. For example, in the embodiment shown in FIG. 10 a transition section 170 has a flexural modulus value greater than that of the distal region 173 but less than that of a proximal section 172.

In at least one embodiment the transition section 170 of the sheath 102 defines a portion of the sheath 102 wherein at least the inner diameter of the sheath necks down or transitions from the greater diameter about the balloon to a lesser diameter about the catheter shaft 144. Though such necking down of the sheathes' inner diameter is not necessary, the transition does provide the sheath 102 with a bias relative to the proximal end of the balloon 114 which may aid in preventing longitudinal displacement of the sheath 102 during advancement of the system 300.

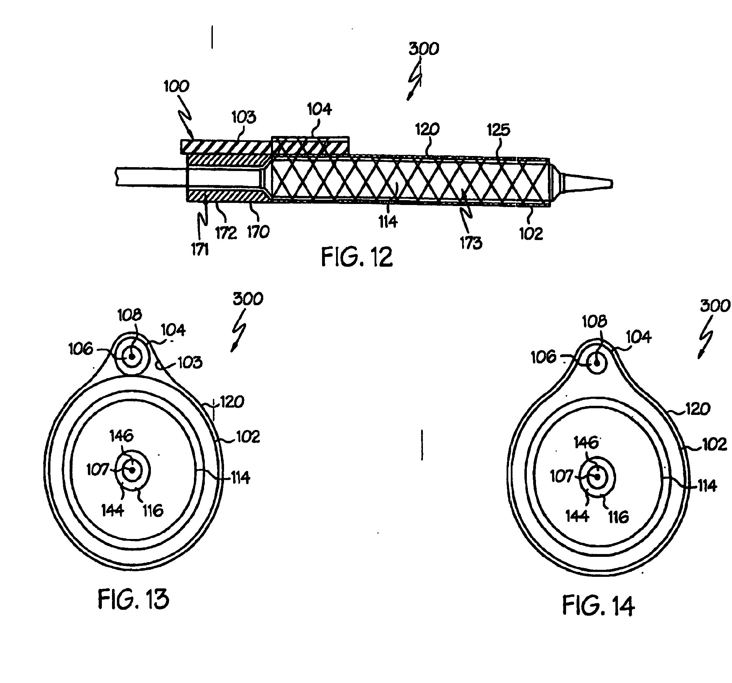

In some embodiments, such as that shown in FIGs. 11 and 12, the sheath 102 may be provided with different regions of stiffness by providing a sheath 102 of a continuous material construction but which has a thinner wall thickness in the distal region 173 than in the proximal region 171. A transition section 170 may be provided where the inner diameter of the sheath 102 is stepped, as in the case of the embodiment shown in FIG. 11, or tapered, as in the case of the embodiment shown in FIG. 12 between the region of the sheath which is disposed about the balloon 114 and the catheter shaft 144.

In some embodiments one or more regions or sections of the sheath 102 may be provided with cuts, slits, indentations or other openings or pores in the wall of the sheath 102 to vary the flexibility and/or stiffness of a respective region or section. Likewise, in some embodiments a coating of a hardening agent or other material(s) may be applied to one or more sections or regions of the sheath 102 in order to modify the hardness, flexibility, and/or stiffness of a respective region or section.

As shown in FIGs. 10-12, the increased length of the sheath 102 provides the assembly 100 with a longer engagement surface is between the sheath 102 and the secondary guidewire housing 104. The secondary guidewire housing 104 may be engaged along a majority or its entire length to the rotatable sheath 104. By providing a more extensive engagement between the housing 104 and sheath 102 the need of a hypotube or other relatively hard outer layer is unnecessary in the construction of the guidewire housing 104 as sufficient stiffness may be provided by at least the proximal region 171 of the sheath 102.

In the embodiments shown in FIGs. 10-12 the housing 104 may be comprised of the relatively flexible inner shaft 103 such as has been described above. The housing 104 may be adhesively or chemically bonded to the sheath 102 and/or may be fused welded or otherwise engaged to the sheath 102 such as in the manner depicted in FIG. 13.

In some embodiments the housing 104 may be integral with the wall of the sheath 102 such as is shown in FIG. 14. In such an embodiment a guidewire opening may be provided radially through the housing 104/sheath 102 in order to allow the secondary guidewire 108 to exit the secondary guidewire lumen 106. In some embodiments the lumen 106 may extend through the length of the sheath 102.

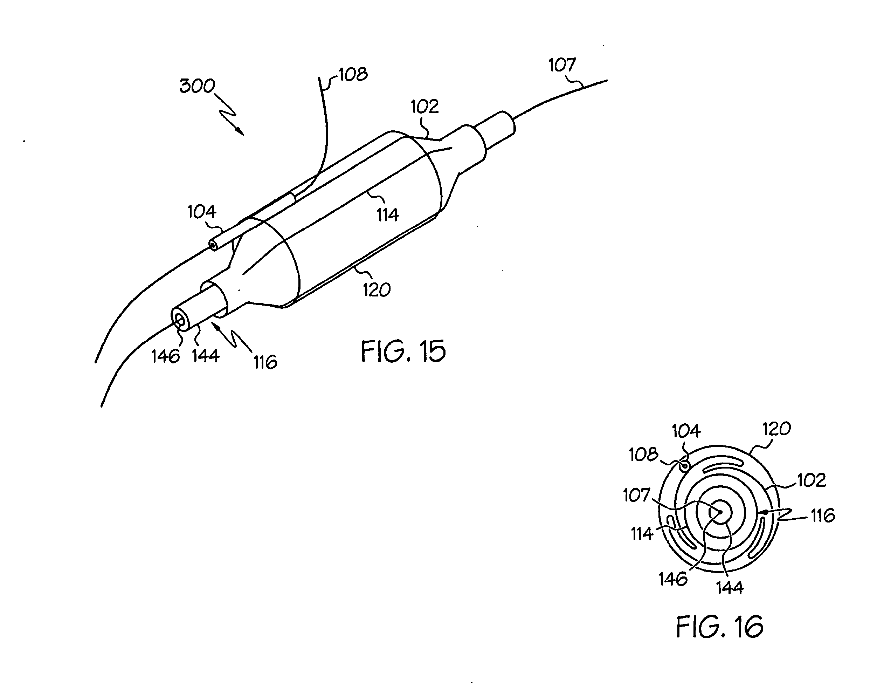

In some embodiments, an example of which is shown in FIG. 15, the rotatable sheath 102 has a length which is about the same as, or somewhat greater than the length of the balloon body 115. In the embodiments shown the sheath 102 is constructed of one or more non-compliant materials whereas the balloon 114 is constructed of one or more compliant materials.

When the balloon 114 is unexpanded during advancement of the system, the sheath 102 is folded or wrapped around the balloon 114, such as in the manner illustrated in FIG. 16. The non-compliant nature of the sheath 102 allows the sheath 102 to be freely rotatable about the balloon when folded thereabout in the folded or "unexpanded" state. When the balloon is expanded, as shown in FIG. 15, the sheath will unfold or unwrap to its nominal unfolded or "expanded" diameter. The non-compliant nature of the sheath 102 allows the nominal diameter of the sheath 102 to be selected in order to limit or alter the expansion of the more compliant balloon 114. In some embodiments, by providing the sheath 102 with tapered end regions the unfolded sheath 102 is biased against the respective cones 117 and 119 of the balloon 114 thereby ensuring that the sheath 102 cannot be substantially longitudinally displaced relative to the balloon 114.

In loading the catheter 116, the non-compliant sheath 102 is slid over the balloon and placed in the folded reduced diameter condition. Once in place the stent 120 is positioned over the sheath 102 and crimped on top of the sheath 102 as well as the secondary guidewire housing 104 if desired. In some embodiments the housing 104 is engaged to the sheath 102 by adhesive, chemical, mechanical, or other form of engagement prior to mounting the stent 120 about the sheath 102 and housing 104.

In some embodiments the non-compliant sheath 102 is constructed of one or more materials including, but not limited to: Nylon 12, Polyethylene terephthalate (PET), polybutylene terephthalate (PBT), Polyamide 12, Polyether block amide (PEbax) 7233^{®}, Pebax 7033^{®}, PTFE, Polyaryletherketones (PEEK), Polyphenylene Oxide (PPO), etc. Other materials include the use reinforcing fibers such as HDPE, stainless steel, and others which may be braided and/or covered by any polymer (non-compliant as well as compliant) as the braiding is providing the non-compliant character.

In some embodiments the compliant balloon 114 is constructed of one or more materials including, but not limited to: silicon rubber, urethane, Polyisobutylene, Polyurethane, SBS, SEBS and SIS, etc.

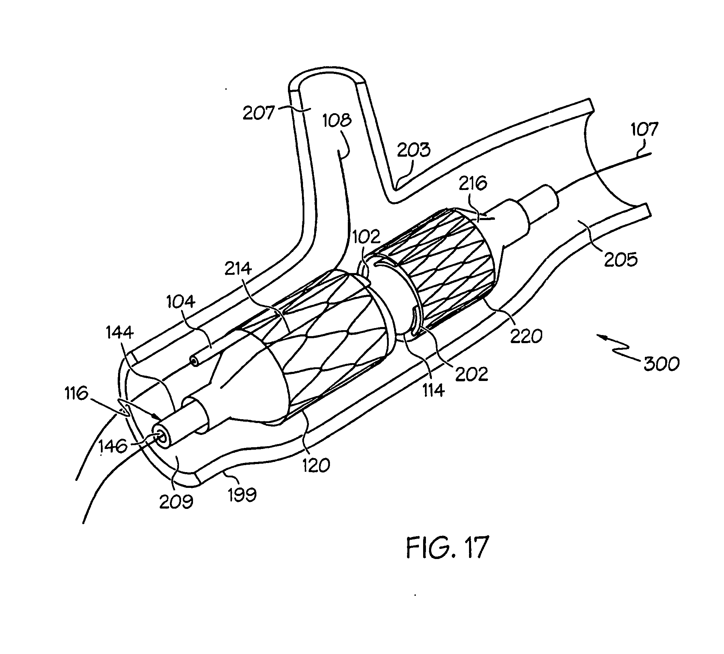

As indicated above the use of non-compliant material or materials in the construction of the rotatable sheath 102 provides the ability to tailor the expansion of the compliant balloon 114. For example, in some embodiments, an example of which is shown in FIG. 17, the system 300 may be configured to deploy two stents 120 and 220 at a vessel bifurcation 203. Because it may be desirable to deploy the first stent 120 into the typically larger diameter main branch 209 of the vessel 199 proximal to the bifurcation 203 and/or at least partially across the opening of a side branch 207, and the second stent 220 into the typically narrower side branch 205 distal of the bifurcation 203, each stent may be disposed about separate non-compliant sheaths 102 and 202. Only one, such as sheath 102, or both sheathes 102 and 202 may be rotatable about the balloon 114. Where only one sheath 102 is rotatable, the other sheath 202 may be engaged by welding, adhesion or other engagement mechanism to the catheter shaft 144 and/or balloon 114.

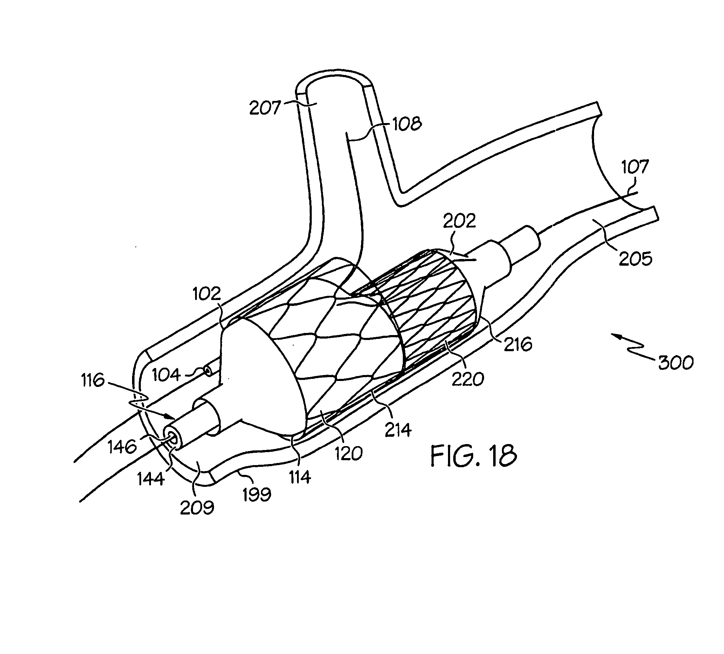

In order to properly deploy the two stents 120 and 220 to a vessel or vessels having different diameters, the balloon 114 must be capable of expanding each sheath and thus each stent to the appropriate extent. Rather than modifying the construction of the balloon, in some embodiments sheaths 102 and 202 are constructed of a substantially non-compliant material, wherein the sheaths have different nominal diameters when the balloon is expanded. Because of their non-compliant nature, the sheaths will limit expansion of the respective portion of the balloon about which they are disposed to the desired nominal diameter of each sheath. For example, in the embodiment shown in FIGs. 17 and 18, the rotatable proximal sheath 102 has a nominal diameter greater than that of the distal sheath 202. As such, when the balloon is expanded to deliver the stents 120 and 220, such as in the manner shown in FIG. 18, the distal region 216 of the balloon 114 will expand only to the extent permitted by the nominal diameter of the distal sheath 202, and the proximal region 214 of the balloon 114 will expand to a greater diameter limited to the nominal diameter of the proximal sheath 102.

As a result, stent 120 is expanded to a greater deployed diameter than the distally positioned stent 220. If desired expansion of the balloon 114 may be controlled by using sheathes of different construction, multiple sheathes, stent configuration, and/or by modifying the expansion characteristics of the balloon and/or catheter. In some embodiments different stents may be expanded or limited to the same or different diameters and to any extent desired in accordance with the concepts described above.

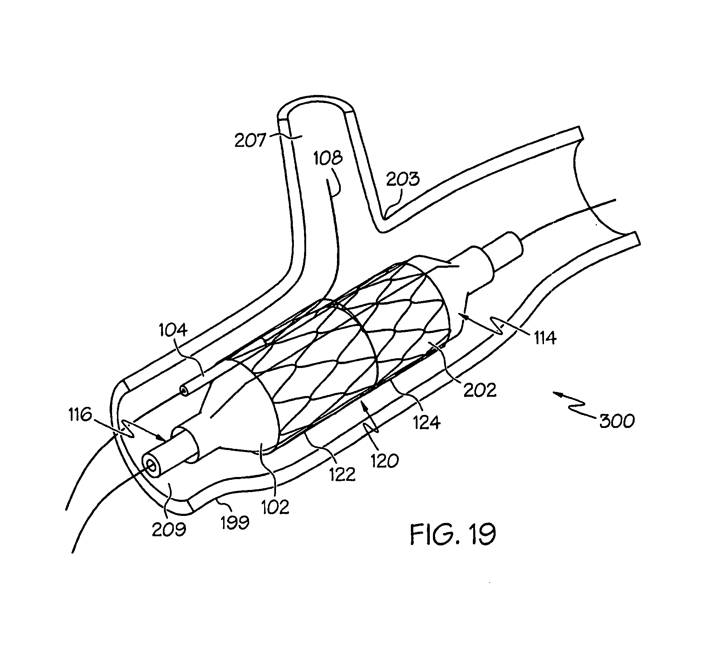

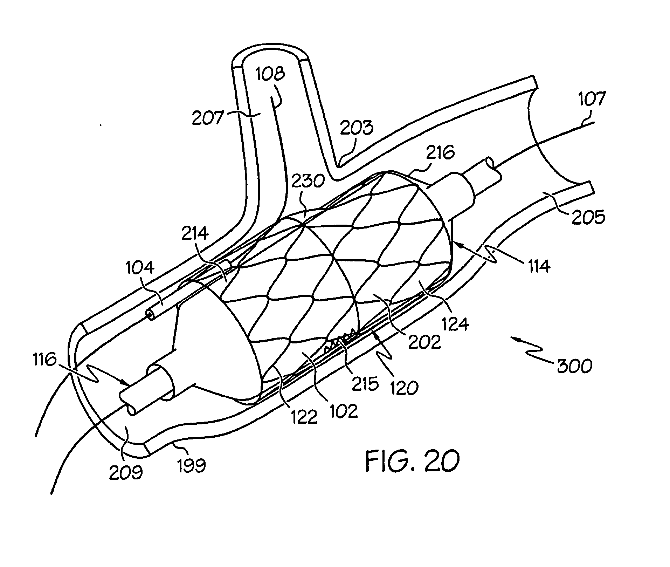

In some embodiments, it may be necessary or desirable to expand a single stent 120 in such a manner that a proximal portion 122 expands to a different diameter than the distal portion 124 such as in the manner shown in FIGs. 19 and 20. In such an instance the stent may be mounted about to rotatable sheathes 102 and 202 of substantially non-compliant construction, wherein one of the sheaths has a nominal diameter greater than that of the other. In the present embodiment, the proximal rotatable sheath 102 has a nominal diameter greater than that of the distal rotatable sheath 202. As a result when the relatively compliant balloon 114 is expanded, the distal region 216 of the balloon, and likewise the distal region 124 of the stent 120, will be limited in expansion by the nominal diameter of the non-compliant distal sheath 202. The proximal region 214 of the balloon 114, and likewise the proximal region 122 of the stent 120, will be expanded to a greater extent than the respective distal regions being limited by the nominal diameter of the substantially non-compliant proximal sheath 102.

As is shown in FIG. 19, at least one of the sheaths 102 and/or 202 may be formed at an angle to provide the sheaths with an overlapping region 215. The sheathes may be independently rotatable prior to delivery or may be engaged to one another at the overlapping region by welding, adhesive engagement, mechanical engagement or other engagement mechanisms. In some embodiments, as a result of the angled configuration of the overlapping sheathes 102 and 202 a region of the sheaths circumferentially adjacent and/or opposite the overlapping region 215 a guidewire gap 230 is defined by the portions of the sheathes that are separated from one another. In some embodiments the presence of the guidewire gap 230 allows the system 300 to be configured with the guidewire housing 104 and/or the guidewire 108 to underlay the proximal sheath 102 and pass radially outward through a secondary stent opening 130a which lies over the gap 230, however as shown in FIGs. 19and 20 the secondary guidewire housing 104 may be positioned on the exterior of the sheath 102 as shown. In some embodiments the guidewire housing may be integral with the construction of the proximal sheath 102 as previously described. In embodiments wherein the housing 104 is positioned under the proximal sheath 102, the housing is configured so as to not substantially interfere with the rotatability of the sheath 102 about the balloon 114.

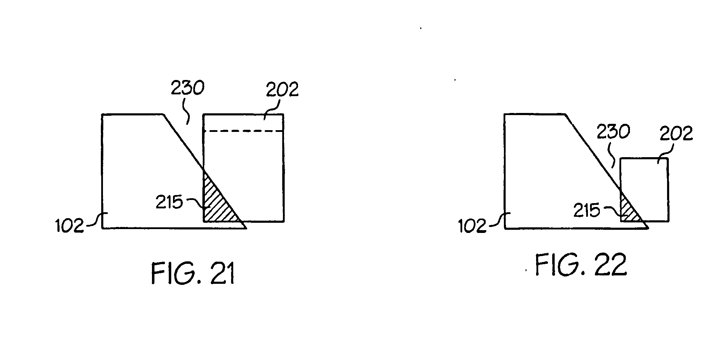

As illustrated in FIGs. 21 and 22 the sheaths 102 and 202 may be configured to overlap to a variety of extents. Also, the nominal diameter of either or both sheathes may be varied.

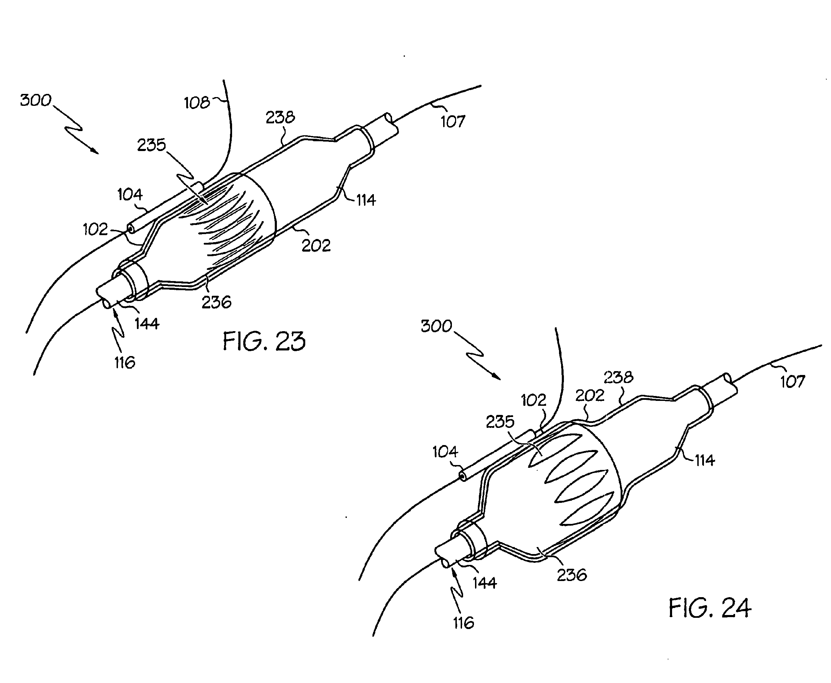

In some embodiments, such as in the example shown in FIGs. 23 and 24, the expansion characteristics of a compliant balloon 114 may be modified by providing the balloon with a cover, sheath or sleeve 202 which has been structurally modified to allow the balloon 114 to expand in one region to a greater extent than in another.

As an initial note, the for illustrative purposes the system 300 depicted in FIGs. 23 and 24 is not shown with a stent or stents thereon. It will be recognized however, that the system 300 shown could of course be utilized with or without a stent or stents as is the case with all of the embodiments of the system 300 described herein.

In the embodiment shown in FIGs. 23 and 24, the balloon cover 202 is a sleeve of substantially non-compliant material which has a length extending over substantially the entire balloon. A region of the cover 202, in this instance the proximal region 236 of the sheath 202, defines a plurality of openings, slits, cuts, pores, thinned areas, etc. 235, through the cover wall. The openings 235 allow the portion of the balloon 114 there under to expand to a greater effective diameter than the portion of the balloon underlying the distal region 238 of the sheath 202 which has no or fewer openings therethrough. As is illustrated in FIGs. 23 and 24, the openings 235 allow the non-compliant sheath to bulge out in the slitted area at the expense of axial shortening.

The balloon cover 202 may be rotatable about or fixedly engaged to the balloon 114 and/or catheter shaft 144 at one or more locations. Rotatably disposed about at least the proximal region 236 of the balloon cover 202 is a rotatable sheath 102 such as has been previously described. In some embodiments a secondary guidewire housing 104 is engaged or is a part of the rotatable sheath 102 such as in any of the manners previously described.

In practice a first stent is mounted about the rotatable sheath 102 and in some embodiments a second stent is disposed about the distal region 238 of the balloon cover 202. As a result of the rotation provided by the rotatable sheath 102 the first stent is independently rotatable about the balloon 114 as the system is advanced through a lumen or vessel. The direction and degree of rotation of the stent and sheath 102 is a consequence of the advancement of the system along the guidewire 108 which has been previously described above in.

Once the system 300 is properly positioned at a vessel bifurcation the compliant balloon 114 is expanded to deliver the stent or stents in the manner previously depicted and described. As the balloon 114 pushes outward against the balloon cover 202, the distal region 238 of the cover 202 will limit the balloons expansion to that of the nominal diameter of the cover 202. The openings 235 in the proximal region 236 of the balloon cover 202 allow the cover 202 to bulge outward in the region of the openings 235 at the expense of axial shortening such as is illustrated in FIGs. 23 and 24. The proximal portion of the balloon may continue expanding until it reaches the limiting nominal diameter of the rotatable sheath 102. As a consequence, stents mounted about the rotatable sheath 102 and/or covering sheath 202 will be expanded to different diameters is indicated by the expansion of the balloon 114 shown in FIG. 24.

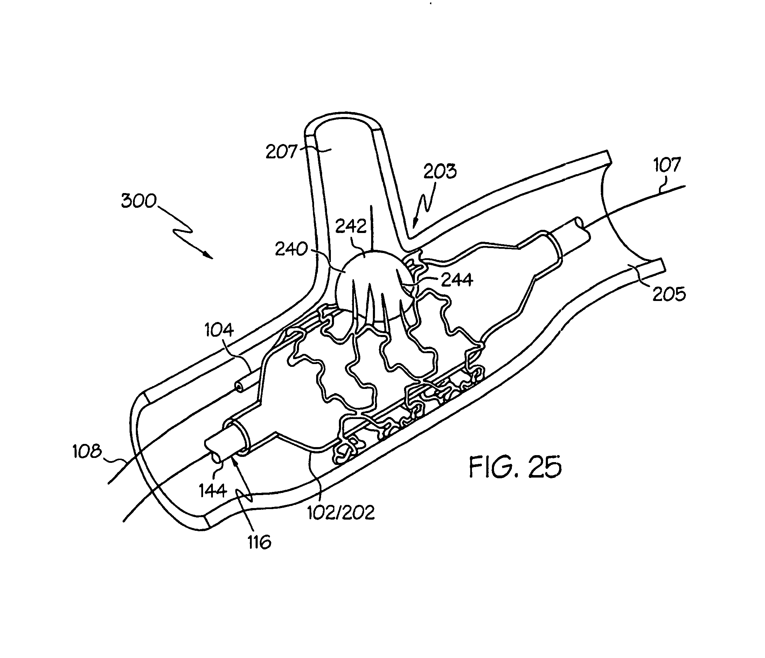

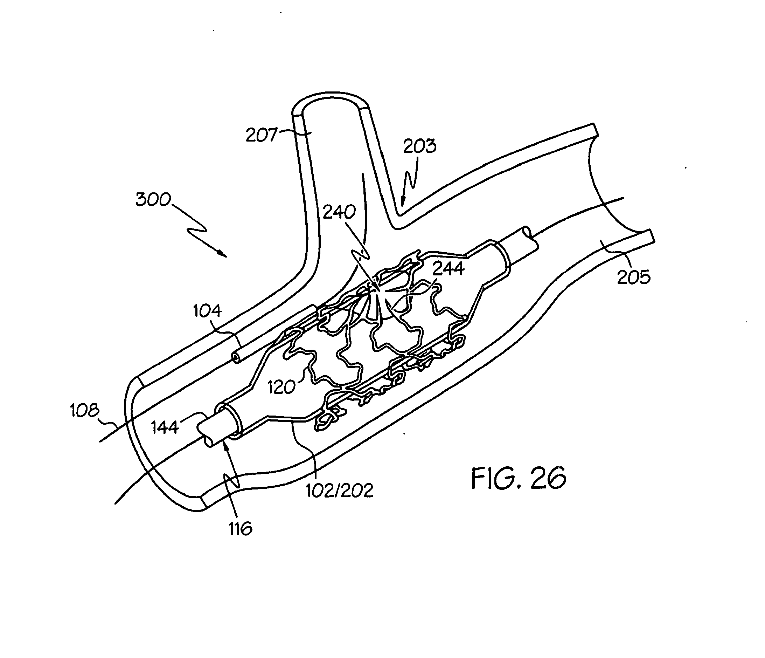

In any of the various embodiments described above, a sheath such as sheath 102 and/or 202 may be provided with an opening, weakened or thinner area, or a predetermined shape which allows the compliant balloon to directly or indirectly deploy a portion of a stent 120, such as a crown region 240 as depicted in FIGs. 25 and 26, into a side branch 207 of a vessel bifurcation 203.

Where the sheath 102 and/or 202 is a non-compliant material the sheath may be provided with a predetermined shape such that in the nominal or expanded diameter a predetermined region or protrusion 242 of the sheath extends radially outward to a greater extent than the rest of the sheath (i.e. protrudes away from the balloon). The protrusion 242 is formed as the expansion of the compliant balloon 114 is directed into the region of the protrusion 242 during balloon inflation. The protrusion 242 will act upon the individual extension members 244 of the crown 240 which otherwise rest substantially within the circumferential plane of the stent as illustrated in FIG. 26, by pushing them radially outward and away from the rest of the stent 120 during expansion. As a result of this pushing action the crown 240 is deployed into the side branch as shown in FIG. 25.

Furthermore, it is noted that the various embodiments shown and described in U.S. Patent Application No. 10/375,689, filed February 27, 2003 and U.S. Patent Application No. 10/657,472, filed September 8, 2003 both of which are entitled *Rotating Balloon Expandable Sheath Bifurcation Delivery*; U.S. Patent Application No. 10/747,546, filed December 29, 2003 and entitled *Rotating Balloon Expandable Sheath Bifurcation Delivery System*; U.S. Patent Application No. 10/757,646, filed January 13, 2004 and entitled *Bifurcated Stent Delivery System*; and U.S. Patent Application No. 10/784,337, filed February 23, 2004 and entitled *Apparatus and Method for Crimping a Stent Assembly* may be utilized with the various embodiments described herein.

The above disclosure is intended to be illustrative and not exhaustive. This description will suggest many variations and alternatives to one of ordinary skill in this art. All these alternatives and variations are intended to be included within the scope of the claims where the term "comprising" means "including, but not limited to". Those familiar with the art may recognize other equivalents to the specific embodiments described herein which equivalents are also intended to be encompassed by the claims.

With this description, those skilled in the art may recognize other equivalents to the specific embodiment described herein. Such equivalents are intended to be encompassed by the claims attached hereto.

## Claims

1. A stent delivery system for alignment in a vessel bifurcation comprising:
a catheter (116), the catheter (116) comprising a catheter shaft (144) and a balloon (114) positioned thereon, the catheter shaft (144) having a shaft length and the balloon (114) having a balloon length, the balloon (114) having an unexpanded state and an expanded state;
a sheath (102), the sheath (102) having a distal region disposed about at least a portion of the balloon (114) and a proximal region disposed about at least a portion of the catheter shaft immediately proximally adjacent to the balloon (114), at least the distal region being expandable from an unexpanded condition to an expanded condition when the balloon (114) is expanded from the unexpanded state to the expanded state, the sheath (102) having a sheath length, the sheath length being greater than the balloon length and less than the catheter shaft length, in the unexpanded condition the sheath (102) being rotatable about the at least a portion of the balloon (114);
a secondary guidewire housing (104), the secondary guidewire housing (104) defining a guidewire lumen (106) for passage of a guidewire therethrough, the secondary guidewire housing (104) having a length, a majority of the length of the secondary guidewire housing (104) being engaged to the sheath (102); and
a stent (120), the stent (120) being expandable from an unexpanded configuration to an expanded configuration, in the unexpanded configuration the stent (120) is disposed about the at least a portion of the sheath disposed about at least a portion of the balloon, wherein at least a portion of the stent (120) in the unexpanded configuration overlies the guidewire housing (104).

2. The stent delivery system of claim 1 wherein the sheath (102) is constructed of a first material and a second material, the first material being less stiff than the second material.

3. The stent delivery system of claim 2 wherein the first material has a flexural modulus value less than a flexural modulus value of the second material.

4. The stent delivery system of claim 2 wherein first material has a flexural modulus value greater than a flexural modulus value of the second material.

5. The stent delivery system of claim 4 wherein the distal region of the sheath (102) is comprised of the first material and at least a portion of the proximal region of the sheath (102) is comprised of the second material.

6. The stent delivery system of claim 5 wherein the sheath (102) is constructed of a third material, the third material being stiffer than the first material and the second material, the proximal region of the sheath having a proximal section and a distal section, the distal section being comprised of the second material and the proximal section being comprised of the third material.

7. The stent delivery system of claim 6 wherein the third material has a flexural modulus greater than the flexural modulus of the first material.

8. The stent delivery system of claim 1 wherein the guidewire housing (104) is at least partially constructed of at least one member of the group consisting of:
Polyisobutylene, Polyurethane, silicone rubber, SBS, SEBS, SIS, latex; stainless steel, nitinol, and any combination thereof.

9. The stent delivery system of claim 1 wherein a distal portion of the guidewire housing (104) extends in a substantially radial direction through a secondary opening of the stent (120).

10. The stent delivery system of claim 1 wherein the guidewire housing (104) is more flexible than at least a portion of the sheath (102).

11. The stent delivery system of claim 1 wherein the sheath (102)defines a sheath wall, at least the majority of the length of the guidewire housing being contained within the sheath wall.

12. The stent delivery system of claim 1 wherein the sheath (102) has a sheath wall thickness, the sheath wall thickness of the distal region of the sheath being less than the sheath wall thickness of the proximal region of the sheath (102).

13. The stent delivery system of claim 12 wherein the sheath wall thickness of the proximal region is about 0.051 mm (0.002 inches) to about 0.508 mm (0.020 inches).

14. The stent delivery system of claim 13 wherein the sheath wall thickness of the distal region is about 0.051 mm (0.002 inches) to about 0.508 mm (0.020 inches).

15. The stent delivery system of claim 12 wherein the sheath (102) has a substantially uniform material construction.

16. The stent delivery system of claim 12 wherein the sheath wall thickness of the proximal region and the sheath wall thickness of the distal region are separated by a step in the sheath wall thickness.

17. The stent delivery system of claim 12 wherein the sheath wall thickness of the proximal region and the sheath wall thickness of the distal region transition from one another by a tapered region of the sheath wall thickness.

18. The stent delivery system of claim 1 wherein the balloon (114) has a proximal region and a distal region, wherein the balloon (114) is constructed of at least one substantially compliant material, wherein each region of the balloon (114) has an unexpanded state and an expanded state, the diameter of a respective region of the balloon (114) being greater in the expanded state than in the unexpanded state.

19. The stent delivery system of claim 18 wherein when the sheath (102) is expanded from the unexpanded condition to the expanded condition, expansion of the sheath (102) is substantially non-elastic.

20. The stent delivery system of claim 19 wherein when the sheath (102) is in the unexpanded condition the sheath (102) is folded about the balloon (114) in the unexpanded state, when the sheath (102) is expanded to the expanded condition the sheath (102) is unfolded from about the balloon (114).

21. The stent delivery system of claim 18 wherein the guidewire housing (104) is at least partially constructed of at least one member of the group consisting of:
Polyisobutylene, Polyurethane, silicone rubber, SBS, SEBS, SIS, latex; stain less steel, nitinol, and any combination thereof.

22. The stent delivery system of claim 18 wherein the guidewire housing (104) is engaged to an inner surface of the sheath (102).

23. The stent delivery system of claim 18 wherein the guidewire housing (104) is engaged to an external surface of the sheath (102).

24. The stent delivery system of claim 18 wherein a distal portion of the guidewire housing (104) extends in a substantially radial direction through a secondary opening of the stent (120).

25. The stent delivery system of claim 24 further comprising a secondary sheath (202), the secondary sheath (202) constructed of at least one substantially non-compliant material, the secondary sheath (202) being expandable from an unexpanded condition to an expanded condition when the balloon (114) is expanded from the unexpanded state to the expanded state, the sheath (102) being rotatably disposed about the proximal region of the balloon (114) and the secondary sheath (202) being disposed about the distal region of the balloon (114).

26. The stent delivery system of claim 25 wherein in the expanded condition the sheath (102) has a diameter greater than the diameter of the secondary sheath (202) in the expanded condition, the diameter of the proximal region of the balloon (114) in the expanded state being limited to be no greater than the diameter of the sheath (102) in the expanded condition, the diameter of the distal region of the balloon (114) in the expanded state being limited to be no greater than the diameter of the secondary sheath (202) in the expanded condition.

27. The stent delivery system of claim 26 further comprising a second stent (220), the second stent (220) being expandable from an unexpanded configuration to an expanded configuration, in the unexpanded configuration the stent is disposed about at least a portion of the secondary sheath (202).

28. The stent delivery system of claim 27 wherein the secondary sheath (202) is rotatable about the distal region of the balloon (114).

29. The stent delivery system of claim 28 wherein the secondary sheath (202) is independently rotatable relative to the sheath (102).

30. The stent delivery system of claim 27 wherein the secondary sheath (202) is fixedly engaged to at least a portion of the catheter (116).

31. The stent delivery system of claim 26 wherein the secondary sheath (202) is rotatable about the distal region of the balloon (114), sheath (102) and the secondary sheath (202) at least partially overlap at an overlapping region.

32. The stent delivery system of claim 30 wherein the sheath (102) and the secondary sheath (202) are fixedly engaged to one another at the overlapping region.

33. The stent delivery system of claim 31 wherein a portion of the sheath (102) and a portion of the secondary sheath (202) are longitudinally separated to define a space, the distal portion of the guidewire housing (104) extends through the space.

34. The stent delivery system of claim 18 wherein the at least one substantially compliant material is selected from at least one member of the group consisting of:
silicone rubber, urethane, Polyisobutylene, Polyurethane, SBS, SEBS, SIS, and any combinations thereof.

35. The stent delivery system of claim 18 wherein the at least one substantially non-compliant material is selected from at least one member of the group consisting of: Nylon 12, PET, PBT, Polyamide 12, Polyether block amide, PTFE, PEEK, PPO, HDPE, stainless steel, and any combinations thereof.

36. The stent delivery system of claim 24 further comprising a balloon cover, the balloon cover disposed about the balloon and underlying at least a portion of the sheath (102), the sheath (102) being rotatably disposed about the balloon cover, the balloon cover being expandable from an unexpanded condition to an expanded condition when the balloon (114) is expanded from the unexpanded state to the expanded state, the balloon cover constructed of at least one substantially non-compliant material.

37. The stent delivery system of claim 36 wherein when the balloon cover is expanded from the unexpanded condition to the expanded condition, expansion of the balloon cover is substantially non-elastic.

38. The stent delivery system of claim 37 wherein the balloon cover comprises a proximal region and a distal region, the proximal region of the balloon cover being disposed about the proximal region of the balloon (114), the distal region of the balloon cover being disposed about the distal region of the balloon (114), the proximal region of the balloon cover defining a plurality of openings therein.

39. The stent delivery system of claim 38 wherein the proximal region of the balloon cover having a diameter in the expanded condition that is greater than a diameter of the distal region of the balloon cover in the expanded condition.

40. The stent delivery system of claim 38 wherein when the balloon (114) is in the expanded state, portions of the proximal region of the balloon expand radially outward through the openings of the proximal region of the balloon cover, the portions of the proximal region of the balloon expanding to a diameter limited by the diameter of the sheath (102) in the expanded condition.

41. The stent delivery system of claim 36 wherein the at least one substantially non-compliant material of the balloon cover is selected from at least one member of the group consisting of: Nylon 12, PET, PBT, Polyamide 12, Polyether block amide, PTFE, PEEK, PPO, HDPE, stainless steel, and any combinations thereof.

42. The stent delivery system of claim 24 wherein the secondary opening of the stent is defined by a crown (240), the crown (240) comprising a plurality of elongate stent members, the crown (240) having an unexpanded position and an expanded position, when the balloon (114) is in the unexpanded state the crown (240) being in the unexpanded position, when the balloon (114) is in the expanded state the crown (240) being in the expanded position, in the unexpanded position the plurality of elongate stent members being contained substantially within the circumference of the stent (120), in the expanded position the plurality of elongate stent members extending radially outward from the circumference of the stent (120).

43. The stent delivery system of claim 42 wherein the sheath (102) defines a weakened area, the weakened area corresponding to the position of the crown in the unexpanded position, in expanded state the weakened area defines a radial protrusion which extends radially outward from a remainder of the sheath (102) to expand the crown to the expanded position.

44. The stent delivery system of claim 18 wherein at least a portion of the stent (120) is coated with at least one therapeutic agent.

45. The stent delivery system of claim 44 wherein the at least one therapeutic agent is at least one non-genetic therapeutic agent selected from at least one member of the group consisting of: anti-thrombogenic agents, genetic material, non-genetic material, cellular material and any combinations thereof.

46. The stent delivery system of claim 44 wherein the at least one therapeutic agent comprises at least one polymer coating.

## Patentansprüche

1. Stentzufuhrsystem zur Ausrichtung in einer Gefäßbifurkation mit:
einem Katheter (116), wobei der Katheter (116) einen Katheterschaft (144) und einen darauf positionierten Ballon (114) aufweist, der Katheterschaft (144) eine Schaftlänge hat und der Ballon (114) eine Ballonlänge hat und der Ballon (114) einen nicht expandierten Zustand und einen expandierten Zustand hat;
einer Hülle (102), wobei die Hülle 102) einen distalen Bereich, der um mindestens einen Abschnitt des Ballons (114) angeordnet ist, und einen proximalen Bereich hat, der um mindestens einen Abschnitt des Katheterschafts unmittelbar proximal benachbart zum Ballon (114) angeordnet ist, mindestens der distale Bereich aus einer nicht expandierten Beschaffenheit in eine expandierte Beschaffenheit expandierbar ist, wenn der Ballon (114) aus dem nicht expandierten Zustand in den expandierten Zustand expandiert wird, die Hülle (102) eine Hüllenlänge hat, die Hüllenlänge größer als die Ballonlänge und kleiner als die Katheterschaftlänge ist und in der nicht expandierten Beschaffenheit die Hülle (102) um den mindestens einen Abschnitt des Ballons (114) drehbar ist; einem sekundären Führungsdrahtgehäuse (104), wobei das sekundäre Führungsdrahtgehäuse (104) ein Führungsdrahtlumen (106) zum Durchgang eines Führungsdrahts bildet, das sekundäre Führungsdrahtgehäuse (104) eine Länge hat und ein Großteil der Länge des sekundären Führungsdrahtgehäuses (104) mit der Hülle (102) im Eingriff steht;
und
einem Stent (120), wobei der Stent (120) aus einer nicht expandierten Konfiguration in eine expandierte Konfiguration expandierbar ist, und in der nicht expandierten Konfiguration der Stent (120) um den mindestens einen Abschnitt der Hülle angeordnet ist, der um mindestens einen Abschnitt des Ballons angeordnet ist, wobei mindestens ein Abschnitt des Stents (120) in der nicht expandierten Konfiguration über dem Führungsdrahtgehäuse (104) liegt.

2. Stentzufuhrsystem nach Anspruch 1, wobei die Hülle (102) aus einem ersten Material und einem zweiten Material aufgebaut ist, wobei das erste Material weniger steif als das zweite Material ist.

3. Stentzufuhrsystem nach Anspruch 2, wobei das erste Material einen Biegemodulwert hat, der kleiner als ein Biegemodulwert des zweiten Materials ist.

4. Stentzufuhrsystem nach Anspruch 2, wobei das erste Material einen Biegemodulwert hat, der größer als ein Biegemodulwert des zweiten Materials ist.

5. Stentzufuhrsystem nach Anspruch 4, wobei der distale Bereich der Hülle (102) das erste Material aufweist und mindestens ein Abschnitt des proximalen Bereichs der Hülle (102) das zweite Material aufweist.

6. Stentzufuhrsystem nach Anspruch 5, wobei die Hülle (102) aus einem dritten Material aufgebaut ist, wobei das dritte Material steifer als das erste Material und das zweite Material ist, der proximale Bereich der Hülle ein proximales Teilstück und ein distales Teilstück hat, das distale Teilstück das zweite Material aufweist und das proximale Teilstück das dritte Material aufweist.

7. Stentzufuhrsystem nach Anspruch 6, wobei das dritte Material einen Biegemodul hat, der größer als der Biegemodul des ersten Materials ist.

8. Stentzufuhrsystem nach Anspruch 1, wobei das Führungsdrahtgehäuse (104) mindestens teilweise aus mindestens einem Element der Gruppe aufgebaut ist, die aus Polyisobutylen, Polyurethan, Silikonkautschuk, SBS, SEBS, SIS, Latex; Edelstahl, Nitinol und jeder Kombination daraus besteht.

9. Stentzufuhrsystem nach Anspruch 1, wobei sich ein distaler Abschnitt des Führungsdrahtgehäuses (104) durch eine sekundäre Öffnung des Stents (120) im Wesentlichen in Radialrichtung erstreckt.

10. Stentzufuhrsystem nach Anspruch 1, wobei das Führungsdrahtgehäuse (104) flexibler als mindestens ein Abschnitt der Hülle (102) ist.

11. Stentzufuhrsystem nach Anspruch 1, wobei die Hülle (102) eine Hüllenwand bildet, wobei mindestens der Großteil der Länge des Führungsdrahtgehäuses in der Hüllenwand enthalten ist.

12. Stentzufuhrsystem nach Anspruch 1, wobei die Hülle (102) eine Hüllenwanddicke hat, wobei die Hüllenwanddicke des distalen Bereichs der Hülle kleiner als die Hüllenwanddicke des proximalen Bereichs der Hülle (102) ist.

13. Stentzufuhrsystem nach Anspruch 12, wobei die Hüllenwanddicke des proximalen Bereichs etwa 0,051 mm (0,002 Inch) bis etwa 0,508 mm (0,020 Inch) beträgt.

14. Stentzufuhrsystem nach Anspruch 13, wobei die Hüllenwanddicke des distalen Bereichs etwa 0,051 mm (0,002 Inch) bis etwa 0,508 mm (0,020 Inch) beträgt.

15. Stentzufuhrsystem nach Anspruch 12, wobei die Hülle (102) einen im Wesentlichen gleichmäßigen Materialaufbau hat.

16. Stentzufuhrsystem nach Anspruch 12, wobei die Hüllenwanddicke des proximalen Bereichs und die Hüllenwanddicke des distalen Bereichs durch eine Stufe in der Hüllenwanddicke getrennt sind.

17. Stentzufuhrsystem nach Anspruch 12, wobei die Hüllenwanddicke des proximalen Bereichs und die Hüllenwanddicke des distalen Bereichs durch einen zulaufenden Bereich der Hüllenwanddicke ineinander übergehen.

18. Stentzufuhrsystem nach Anspruch 1, wobei der Ballon (114) einen proximalen Bereich und einen distalen Bereich hat, wobei der Ballon (114) aus mindestens einem im Wesentlichen nachgiebigen Material aufgebaut ist, wobei jeder Bereich des Ballons (114) einen nicht expandierten Zustand und einen expandierten Zustand hat, wobei der Durchmesser eines jeweiligen Bereichs des Ballons (114) im expandierten Zustand größer als im nicht expandierten Zustand ist.

19. Stentzufuhrsystem nach Anspruch 18, wobei bei Expansion der Hülle (102) aus der nicht expandierten Beschaffenheit in die expandierte Beschaffenheit die Expansion der Hülle (102) im Wesentlichen unelastisch ist.

20. Stentzufuhrsystem nach Anspruch 19, wobei in der nicht expandierten Beschaffenheit der Hülle (102) die Hülle (102) um den Ballon (114) im nicht expandierten Zustand gefaltet ist und bei Expansion der Hülle (102) in die expandierte Beschaffenheit die Hülle (102) aus ihrer Lage um den Ballon (114) entfaltet wird.

21. Stentzufuhrsystem nach Anspruch 18, wobei das Führungsdrahtgehäuse (104) mindestens teilweise aus mindestens einem Element der Gruppe aufgebaut ist, die aus Polyisobutylen, Polyurethan, Silikonkautschuk, SBS, SEBS, SIS, Latex; Edelstahl, Nitinol und jeder Kombination daraus besteht.

22. Stentzufuhrsystem nach Anspruch 18, wobei das Führungsdrahtgehäuse (104) mit einer Innenfläche der Hülle (102) im Eingriff steht.

23. Stentzufuhrsystem nach Anspruch 18, wobei das Führungsdrahtgehäuse (104) mit einer Außenfläche der Hülle (102) im Eingriff steht.

24. Stentzufuhrsystem nach Anspruch 18, wobei sich ein distaler Abschnitt des Führungsdrahtgehäuses (104) durch eine sekundäre Öffnung des Stents (120) im Wesentlichen in Radialrichtung erstreckt.

25. Stentzufuhrsystem nach Anspruch 24, ferner mit einer sekundären Hülle (202), wobei die sekundäre Hülle (202) aus mindestens einem im Wesentlichen unnachgiebigen Material aufgebaut ist, die sekundäre Hülle (202) aus einer nicht expandierten Beschaffenheit in eine expandierte Beschaffenheit expandierbar ist, wenn der Ballon (114) aus dem nicht expandierten Zustand in den expandierten Zustand expandiert wird, die Hülle (102) um den proximalen Bereich des Ballons (114) drehbar angeordnet ist und die sekundäre Hülle (202) um den distalen Bereich des Ballons (114) angeordnet ist.

26. Stentzufuhrsystem nach Anspruch 25, wobei in der expandierten Beschaffenheit die Hülle (102) einen größeren Durchmesser als der Durchmesser der sekundären Hülle (202) in der expandierten Beschaffenheit hat, wobei der Durchmesser des proximalen Bereichs des Ballons (114) im expandierten Zustand so begrenzt ist, dass er nicht größer als der Durchmesser der Hülle (102) in der expandierten Beschaffenheit ist, und der Durchmesser des distalen Bereichs des Ballons (114) im expandierten Zustand so begrenzt ist, dass er nicht größer als der Durchmesser der sekundären Hülle (202) in der expandierten Beschaffenheit ist.

27. Stentzufuhrsystem nach Anspruch 26, ferner mit einem zweiten Stent (220), wobei der zweite Stent (220) aus einer nicht expandierten Konfiguration in eine expandierte Konfiguration expandierbar ist und in der nicht expandierten Konfiguration der Stent um mindestens einen Abschnitt der sekundären Hülle (202) angeordnet ist.

28. Stentzufuhrsystem nach Anspruch 27, wobei die sekundäre Hülle (202) um den distalen Bereich des Ballons (114) drehbar ist.

29. Stentzufuhrsystem nach Anspruch 28, wobei die sekundäre Hülle (202) relativ zur Hülle (102) unabhängig drehbar ist.

30. Stentzufuhrsystem nach Anspruch 27, wobei die sekundäre Hülle (202) mit mindestens einem Abschnitt des Katheters (116) fest im Eingriff steht.

31. Stentzufuhrsystem nach Anspruch 26, wobei die sekundäre Hülle (202) um den distalen Bereich des Ballons (114) drehbar ist und sich die Hülle (102) und die sekundäre Hülle (202) in einem Überlappungsbereich mindestens teilweise überlappen.

32. Stentzufuhrsystem nach Anspruch 30, wobei die Hülle (102) und die sekundäre Hülle (202) im Überlappungsbereich fest im Eingriff miteinander stehen.

33. Stentzufuhrsystem nach Anspruch 31, wobei ein Abschnitt der Hülle (102) und ein Abschnitt der sekundären Hülle (202) in Längsrichtung getrennt sind, um einen Raum zu bilden, wobei sich der distale Abschnitt des Führungsdrahtgehäuses (104) durch den Raum erstreckt.

34. Stentzufuhrsystem nach Anspruch 18, wobei das mindestens eine im Wesentlichen nachgiebige Material aus mindestens einem Element der Gruppe ausgewählt ist, die aus Silikonkautschuk, Urethan, Polyisobutylen, Polyurethan, SBS, SEBS, SIS und allen Kombinationen daraus besteht.

35. Stentzufuhrsystem nach Anspruch 18, wobei das mindestens eine im Wesentlichen unnachgiebige Material aus mindestens einem Element der Gruppe ausgewählt ist, die aus Nylon 12, PET, PBT, Polyamid 12, Polyetherblockamid, PTFE, PEEK, PPO, HDPE, Edelstahl und allen Kombinationen daraus besteht.

36. Stentzufuhrsystem nach Anspruch 24, ferner mit einer Ballonabdeckung, wobei die Ballonabdeckung um den Ballon angeordnet ist und unter mindestens einem Abschnitt der Hülle (102) liegt, die Hülle (102) um die Ballonabdeckung drehbar angeordnet ist, die Ballonabdeckung aus einer nicht expandierten Beschaffenheit in eine expandierte Beschaffenheit expandierbar ist, wenn der Ballon (114) aus dem nicht expandierten Zustand in den expandierten Zustand expandiert wird, und die Ballonabdeckung aus mindestens einem im Wesentlichen unnachgiebigen Material aufgebaut ist.

37. Stentzufuhrsystem nach Anspruch 36, wobei bei Expansion der Ballonabdeckung aus der nicht expandierten Beschaffenheit in die expandierte Beschaffenheit die Expansion der Ballonabdeckung im Wesentlichen unelastisch ist.

38. Stentzufuhrsystem nach Anspruch 37, wobei die Ballonabdeckung einen proximalen Bereich und einen distalen Bereich aufweist, wobei der proximale Bereich der Ballonabdeckung um den proximalen Bereich des Ballons (114) angeordnet ist, der distale Bereich der Ballonabdeckung um den distalen Bereich des Ballons (114) angeordnet ist und der proximale Bereich der Ballonabdeckung mehrere Öffnungen darin bildet.

39. Stentzufuhrsystem nach Anspruch 38, wobei der proximale Bereich der Ballonabdeckung einen Durchmesser in der expandierten Beschaffenheit hat, der größer als ein Durchmesser des distalen Bereichs der Ballonabdeckung in der expandierten Beschaffenheit ist.

40. Stentzufuhrsystem nach Anspruch 38, wobei im expandierten Zustand des Ballons (114) Abschnitte des proximalen Bereichs des Ballons durch die Öffnungen des proximalen Bereichs der Ballonabdeckung radial nach außen expandieren, wobei die Abschnitte des proximalen Bereichs des Ballons auf einen Durchmesser expandieren, der durch den Durchmesser der Hülle (102) in der expandierten Beschaffenheit begrenzt ist.

41. Stentzufuhrsystem nach Anspruch 36, wobei das mindestens eine im Wesentlichen unnachgiebige Material der Ballonabdeckung aus mindestens einem Element der Gruppe ausgewählt ist, die aus Nylon 12, PET, PBT, Polyamid 12, Polyetherblockamid, PTFE, PEEK, PPO, HDPE, Edelstahl und allen Kombinationen daraus besteht.

42. Stentzufuhrsystem nach Anspruch 24, wobei die sekundäre Öffnung des Stents durch eine Krone (240) gebildet ist, wobei die Krone (240) mehrere längliche Stentelemente aufweist, die Krone (240) eine nicht expandierte Position und eine expandierte Position hat, im nicht expandierten Zustand des Ballons (114) sich die Krone (240) in der nicht expandierten Position befindet, im expandierten Zustand des Ballons (114) sich die Krone (240) in der expandierten Position befindet, in der nicht expandierten Position die mehreren länglichen Stentelemente im Wesentlichen im Umfang des Stents (120) enthalten sind und in der expandierten Position sich die mehreren länglichen Stentelemente vom Umfang des Stents (120) radial nach außen erstrecken.

43. Stentzufuhrsystem nach Anspruch 42, wobei die Hülle (102) ein geschwächtes Gebiet bildet, wobei das geschwächte Gebiet der Position der Krone in der nicht expandierten Position entspricht, und im expandierten Zustand das geschwächte Gebiet einen Radialvorsprung bildet, der sich von einem Rest der Hülle (102) radial nach außen erstreckt, um die Krone in die expandierte Position zu expandieren.

44. Stentzufuhrsystem nach Anspruch 18, wobei mindestens ein Abschnitt des Stents (120) mit mindestens einem Therapeutikum beschichtet ist.

45. Stentzufuhrsystem nach Anspruch 44, wobei das mindestens eine Therapeutikum mindestens ein nicht genetisches Therapeutikum ist, das aus mindestens einem Element der Gruppe ausgewählt ist, die aus Antithrombotika, genetischem Material, nicht genetischem Material, Zellmaterial und allen Kombinationen daraus besteht.

46. Stentzufuhrsystem nach Anspruch 44, wobei das mindestens eine Therapeutikum mindestens eine Polymerbeschichtung aufweist.

## Revendications

1. Système de pose de stent pour l'alignement dans une bifurcation de vaisseau, comprenant :
un cathéter (116), le cathéter (116) comprenant une tige de cathéter (144) et un ballonnet (114) positionné sur cette dernière, la tige de cathéter (144) ayant une longueur de tige et le ballonnet (114) ayant une longueur de ballonnet, le ballonnet (114) ayant un état non expansé et un état expansé ;
une gaine (102), la gaine (102) ayant une région distale disposée autour d'au moins une partie du ballonnet (114) et une région proximale disposée autour d'au moins une partie de la tige de cathéter positionnée de manière immédiatement adjacente du point de vue proximal par rapport au ballonnet (114), au moins la région distale étant expansible d'une condition non expansée à une condition expansée lorsque le ballonnet (114) est expansé de l'état non expansé à l'état expansé, la gaine (102) ayant une longueur de gaine, la longueur de gaine étant supérieure à la longueur de ballonnet et inférieure à la longueur de tige de cathéter, dans la condition non expansée, la gaine (102) pouvant tourner autour d'au moins une partie du ballonnet (114) ;
un boîtier de fil guide secondaire (104), le boîtier de fil guide secondaire (104) définissant une lumière de fil guide (106) pour le passage d'un fil guide à travers cette dernière, le boîtier de fil guide secondaire (104) ayant une longueur, une majeure partie de la longueur du boîtier de fil guide secondaire (104) étant mise en prise sur la gaine (102) ; et
un stent (120), le stent (120) étant expansible d'une configuration non expansée à une configuration expansée, dans la configuration non expansée, le stent (120) est disposé autour de la au moins une partie de la gaine disposée autour d'au moins une partie du ballonnet, dans lequel au moins une partie du stent (120) dans la configuration non expansée, recouvre le boîtier de fil guide (104).

2. Système de pose de stent selon la revendication 1, dans lequel la gaine (102) est fabriquée avec un premier matériau et un deuxième matériau, le premier matériau étant moins rigide que le deuxième matériau.

3. Système de pose de stent selon la revendication 2, dans lequel le premier matériau a une valeur de module de flexion inférieure à une valeur de module de flexion du deuxième matériau.

4. Système de pose de stent selon la revendication 2, dans lequel le premier matériau a une valeur de module de flexion supérieure à une valeur de module de flexion du deuxième matériau.

5. Système de pose de stent selon la revendication 4, dans lequel la région distale de la gaine (102) est composée du premier matériau et au moins une partie de la région proximale de la gaine (102) est composée du deuxième matériau.

6. Système de pose de stent selon la revendication 5, dans lequel la gaine (102) est fabriquée avec un troisième matériau, le troisième matériau étant plus rigide que le premier matériau et que le deuxième matériau, la région proximale de la gaine ayant une section proximale et une section distale, la section distale étant composée du deuxième matériau et la section proximale étant composée du troisième matériau.

7. Système de pose de stent selon la revendication 6, dans lequel le troisième matériau a un module de flexion supérieur au module de flexion du premier matériau.

8. Système de pose de stent selon la revendication 1 dans lequel le boîtier de fil guide (104) est au moins partiellement fabriqué avec au moins un élément du groupe comprenant : le polyisobutylène, le polyuréthane, le caoutchouc de silicone, SBS, SEBS, SIS, le latex ; l'acier inoxydable, le Nitinol et l'une quelconque de leurs combinaisons.

9. Système de pose de stent selon la revendication 1, dans lequel une partie distale du boîtier de fil guide (104) s'étend dans une direction sensiblement radiale à travers une ouverture secondaire du stent (120).

10. Système de pose de stent selon la revendication 1, dans lequel le boîtier de fil guide (104) est plus flexible qu'au moins une partie de la gaine (102).

11. Système de pose de stent selon la revendication 1, dans lequel la gaine (102) définit une paroi de gaine, au moins la majeure partie de la longueur du boîtier de fil guide étant contenue à l'intérieur de la paroi de gaine.

12. Système de pose de stent selon la revendication 1, dans lequel la gaine (102) a une épaisseur de paroi de gaine, l'épaisseur de paroi de gaine de la région distale de la gaine étant inférieure à l'épaisseur de paroi de gaine de la région proximale de la gaine (102).

13. Système de pose de stent selon la revendication 12, dans lequel l'épaisseur de paroi de gaine de la région proximale est de l'ordre d'environ 0,051 mm (0,002 pouces) à environ 0,508 mm (0,020 pouces).

14. Système de pose de stent selon la revendication 13, dans lequel l'épaisseur de paroi de gaine de la région distale est de l'ordre d'environ 0,051 mm (0,002 pouces) à environ 0,508 mm (0,020 pouces).

15. Système de pose de stent selon la revendication 12, dans lequel la gaine (102) a une construction de matériau sensiblement uniforme.

16. Système de pose de stent selon la revendication 12, dans lequel l'épaisseur de paroi de gaine de la région proximale et l'épaisseur de paroi de gaine de la région distale sont séparées par un échelon dans l'épaisseur de paroi de gaine.

17. Système de pose de stent selon la revendication 12, dans lequel l'épaisseur de paroi de gaine de la région proximale et l'épaisseur de paroi de gaine de la région distale effectuent un mouvement de transition l'une par rapport à l'autre grâce à une région progressivement rétrécie de l'épaisseur de paroi de gaine.

18. Système de pose de stent selon la revendication 1, dans lequel le ballonnet (114) a une région proximale et une région distale, dans lequel le ballonnet (114) est fabriqué avec au moins un matériau sensiblement souple, dans lequel chaque région du ballonnet (114) a un état non expansé et un état expansé, le diamètre d'une région respective du ballonnet (114) étant plus important à l'état expansé qu'à l'état non expansé.

19. Système de pose de stent selon la revendication 18, dans lequel lorsque la gaine (102) est expansée de la condition non expansée à la condition expansée, l'expansion de la gaine (102) est sensiblement non élastique.

20. Système de pose de stent selon la revendication 19, dans lequel lorsque la gaine (102) est dans la condition non expansée, la gaine (102) est pliée autour du ballonnet (114) à l'état non expansé, lorsque la gaine (102) est expansée dans la condition expansée, la gaine (102) n'est pas pliée autour du ballonnet (114).

21. Système de pose de stent selon la revendication 18, dans lequel le boîtier de fil guide (104) est au moins partiellement fabriqué avec au moins un élément du groupe comprenant : le polyisobutylène, le polyuréthane, le caoutchouc de silicone, SBS, SEBS, SIS, le latex ; l'acier inoxydable, le Nitinol et l'une quelconque de leurs combinaisons.

22. Système de pose de stent selon la revendication 18, dans lequel le boîtier de fil guide (104) est mis en prise sur une surface interne de la gaine (102).

23. Système de pose de stent selon la revendication 18, dans lequel le boîtier de fil guide (104) est mis en prise sur une surface externe de la gaine (102).

24. Système de pose de stent selon la revendication 18, dans lequel une partie distale du boîtier de fil guide (104) s'étend dans une direction sensiblement radiale en passant par une ouverture secondaire du stent (120).

25. Système de pose de stent selon la revendication 24 comprenant en outre une gaine secondaire (202), la gaine secondaire (202) étant fabriquée à partir d'au moins un matériau sensiblement rigide, la gaine secondaire (202) étant expansible d'une condition non expansée à une condition expansée lorsque le ballonnet (114) est expansé de l'état non expansé à l'état expansé, la gaine (102) étant disposée de manière rotative autour de la région proximale du ballonnet (114) et la gaine secondaire (202) étant disposée autour de la région distale du ballonnet (114).

26. Système de pose de stent selon la revendication 25, dans lequel, dans la condition expansée, la gaine (102) a un diamètre supérieur au diamètre de la gaine secondaire (202) dans la condition expansée, le diamètre de la région proximale du ballonnet (114) à l'état expansé étant limité pour ne pas être supérieur au diamètre de la gaine (102) dans la condition expansée, le diamètre de la région distale du ballonnet (114) à l'état expansé étant limité pour ne pas être supérieur au diamètre de la gaine secondaire (202) dans la condition expansée.

27. Système de pose de stent selon la revendication 26, comprenant en outre un deuxième stent (220), le deuxième stent (220) étant expansible d'une configuration non expansée à une configuration expansée, dans la configuration non expansée, le stent est disposé autour d'au moins une partie de la gaine secondaire (202).

28. Système de pose de stent selon la revendication 27, dans lequel la gaine secondaire (202) peut tourner autour de la région distale du ballonnet (114).

29. Système de pose de stent selon la revendication 28, dans lequel la gaine secondaire (202) peut tourner de manière indépendante par rapport à la gaine (102).

30. Système de pose de stent selon la revendication 27, dans lequel la gaine secondaire (202) est mise en prise de manière fixe sur au moins une partie du cathéter (116).

31. Système de pose de stent selon la revendication 26, dans lequel la gaine secondaire (202) peut tourner autour de la région distale du ballonnet (114), la gaine (102) et la gaine secondaire (202) se chevauchent au moins partiellement au niveau d'une région de chevauchement.

32. Système de pose de stent selon la revendication 30, dans lequel la gaine (102) et la gaine secondaire (202) sont mises en prise de manière fixe l'une par rapport à l'autre au niveau de la région de chevauchement.

33. Système de pose de stent selon la revendication 31, dans lequel une partie de la gaine (102) et une partie de la gaine secondaire (202) sont séparées longitudinalement pour définir un espace, la partie distale du boîtier de fil guide (104) s'étend à travers l'espace.

34. Système de pose de stent selon la revendication 18, dans lequel le au moins un matériau sensiblement souple est choisi parmi au moins un élément du groupe comprenant : le caoutchouc de silicone, l'uréthane, le polyisobutylène, le polyuréthane, SBS, SEBS, SIS et l'une quelconque de leurs combinaisons.

35. Système de pose de stent selon la revendication 18, dans lequel le au moins un matériau sensiblement rigide est choisi à partir d'un élément du groupe comprenant : le Nylon 12, PET, PBT, le polyamide 12, le polyéther bloc-amide, PTFE, PEEK, PPO, HDPE, l'acier inoxydable et l'une quelconque de leurs combinaisons.

36. Système de pose de stent selon la revendication 24, comprenant en outre un couvercle de ballonnet, le couvercle de ballonnet étant disposé autour du ballonnet et au-dessous d'au moins une partie de la gaine (102), la gaine (102) étant disposée de manière rotative autour du couvercle de ballonnet, le couvercle de ballonnet étant expansible d'une condition non expansée à une condition expansée lorsque le ballonnet (114) est expansé de l'état non expansé à l'état expansé, le couvercle de ballonnet étant construit avec au moins un matériau sensiblement rigide.

37. Système de pose de stent selon la revendication 36, dans lequel lorsque le couvercle de ballonnet est expansé de la condition non expansée à la condition expansée, l'expansion du couvercle de ballonnet est sensiblement non élastique.

38. Système de pose de stent selon la revendication 37, dans lequel le couvercle de ballonnet comprend une région proximale et une région distale, la région proximale du couvercle de ballonnet étant disposée autour de la région proximale du ballonnet (114), la région distale du couvercle de ballonnet étant disposée autour de la région distale du ballonnet (114), la région proximale du couvercle de ballonnet définissant une pluralité d'ouvertures à l'intérieur de cette dernière.

39. Système de pose de stent selon la revendication 38, dans lequel la région proximale du couvercle de ballonnet ayant un diamètre dans la condition expansée qui est supérieur à un diamètre de la région distale du couvercle de ballonnet dans la condition expansée.

40. Système de pose de stent selon la revendication 38, dans lequel lorsque le ballonnet (114) est à l'état expansé, des parties de la région proximale du ballonnet s'expansent radialement vers l'extérieur à travers les ouvertures de la région proximale du couvercle de ballonnet, les parties de la région proximale du ballonnet s'expansant jusqu'à un diamètre limité par le diamètre de la gaine (102) dans la condition expansée.

41. Système de pose de stent selon la revendication 36, dans lequel le au moins un matériau sensiblement rigide du couvercle de ballonnet est choisi parmi au moins un élément du groupe comprenant : le Nylon 12, PET, PBT, le polyamide 12, le polyéther bloc-amide, PTFE, PEEK, PPO, HDPE, l'acier inoxydable et l'une quelconque de leurs combinaisons.

42. Système de pose de stent selon la revendication 24, dans lequel l'ouverture secondaire du stent est définie par une couronne (240), la couronne (240) comprenant une pluralité d'éléments de stent allongés, la couronne (240) ayant une position non expansée et une position expansée, lorsque le ballonnet (114) est à l'état non expansé, la couronne (240) étant dans la position non expansée, lorsque le ballonnet (114) est à l'état expansé, la couronne (240) étant dans la position expansée, dans la position non expansée, la pluralité des éléments de stent allongés étant contenus sensiblement à l'intérieur de la circonférence du stent (120), dans la position expansée, la pluralité d'éléments de stent allongés s'étendant radialement vers l'extérieur à partir de la circonférence du stent (120).

43. Système de pose de stent selon la revendication 42, dans lequel la gaine (102) définit une zone affaiblie, la zone affaiblie correspondant à la position de la couronne dans la position non expansée, à l'état expansé, la zone affaiblie définit une saillie radiale qui s'étend radialement vers l'extérieur à partir d'un reste de la gaine (102) afin d'expanser la couronne dans la position expansée.

44. Système de pose de stent selon la revendication 18, dans lequel au moins une partie du stent (120) est recouverte avec au moins un agent thérapeutique.

45. Système de pose de stent selon la revendication 44, dans lequel le au moins un agent thérapeutique est au moins un agent thérapeutique non génétique choisi parmi au moins un élément du groupe comprenant : les agents anti-thrombogène, une matière génétique, une matière non génétique, une matière cellulaire et l'une quelconque de leurs combinaisons.

46. Système de pose de stent selon la revendication 44, dans lequel le au moins un agent thérapeutique comprend au moins un revêtement polymère.
